# EUROPÄISCHE PATENTSCHRIFT

(11) **EP 0 535 001 B1**
(45) Veröffentlichungstag und Bekanntmachung des Hinweises auf die Patenterteilung: **24.08.1994**
(21) Anmeldenummer: 91909678.4
(22) Anmeldetag: 27.05.1991
(51) Int. Cl.: A61K 35/78, A61K 35/10, C08H 5/02

(54) **MOLEKÜLVERBUNDSYSTEM ZUR KONTRA-ESKALATIVEN THERAPIE VIRALER INFEKTIONSKRANKHEITEN**
COMPOSITE MOLECULAR SYSTEM FOR THE CONTRA-ESCALATORY TREATMENT OF VIRAL INFECTIONS
SYSTEME COMPOSITE MOLECULAIRE POUR LE TRAITEMENT CONTRE-ESCALATIF DE MALADIES INFECTIEUSES VIRALES

(30) Priorität: 27.05.1990 DE 4017091
(43) Veröffentlichungstag der Anmeldung: 07.04.1993
(73) Patentinhaber: Mach, Chantal, D-85614 Kirchseeon (DE)
(72) Erfinder: Mach, Chantal, D-85614 Kirchseeon (DE)
(74) Vertreter: Neidl-Stippler, Cornelia, Dr.
(86) Internationale Anmeldenummer: DE9100450
(87) Internationale Veröffentlichungsnummer: WO9118616

(56) Entgegenhaltungen:
- EP-A- 296 626
- EP-A- 0 281 679
- ENVIRONMENTAL RESEARCH, Vol. 32, No. 2. Dec. 1983, pages 329 - 343, Academic Press Inc.; see page 333, lines 18 - 23
- WPIL, FILE SUPPLIER; AN=91-012374, Derwent Publications Ltd, (London, GB);

## Beschreibung

Die Erfindung betrifft eine Wirkstoffgruppe zur "restitutiven Chemotherapie" bei viralen Infektionskrankheiten, sowie dessen Verwendung.

Die Bekämpfung von Virus-Infektionen ist ein bisher noch unvollkommen gelöstes Problem. Eines der ungelösten Probleme ist die relativ rasche Mutation von Viren, die die Wirkung von antiviralen Arzneistoffen oder Körpereigenen Antikörpern ganz oder teilweise verhindert. Eine Methode, gegen multi-virale Infektionen verschiedenster Art widerstandsfähig zu machen, besteht darin, einen "Wirkstoffcocktail" einzusetzen, wobei einer der Wirkstoffe wahrscheinlich wirksam sein wird - dieses Prinzip ist von der Fa. SANDOZ als Gammaglobulinpräparat unter dem Warenzeichen "Sandoglobulin" erhältlich - es handelt sich dabei um ein Gemisch von Antikörpern aus dem Blut von mehr als 2000 mitteleuropäischen Spendern, die einen großen Teil des Spektrums von Antikörpern gegen Erkrankungen im mitteleuropäischen Raum aufweisen und daher angenommen wird, daß dieses Produkt gegen verschiedenste Infektionen aufgrund der Vielzahl ver-schiedener Antikörper wirkt.

Bisher wurden Versuche der Virusinfektionsbekämpfung durchgeführt, die an verschiedenen Punkten der Virusreplikation angreifen. So gibt es Medikamente vom Typ Azidothymidin (AZT), die die reverse Transkription durch Kettenabbruch hemmen - diese Medikamente führen aber auch in unerwünschter Weise zu erheblichen Nebenwirkungen im Patienten.

Weltweite Untersuchungen mit Dextransulfat (Verbindungen mit ca 10 kD) aus pflanzlichen Hemizellulosen haben gezeigt, daß auch diese Stoffgruppe zur Therapie von HIV-Patienten keinen praktischen Fortschritt bedeutet.

Die orale Medikation mit Dextransulfat leidet u.a. darunter, daß die Wege des Dextrans in der Blutbahn und die Konzentration am Ort des Kontaktes mit den Viren nicht sehr effektiv sind und daß es gravierende Nebenwirkungen (Neuropenie, Diarrhoe) besitzt. Auch klinisch konnten keine eindrucksvollen Wirkungen festgestellt werden.

Es wurde bereits versucht, isolierte CD4-Rezeptorproteine gentechnologisch herzustellen, die sich fest an das Virus gp 120 binden sollten und demzufolge durch Absättigung desselben das Virus neutralisieren und inaktivieren sollten. Um dieses künstliche CD4 als Therapeutikum einzusetzen, wäre es jedoch nötig, dieses künstliche Protein in hohen Dosen zu injizieren. Die künstliche hohe Überkonzentration eines Zellsensors, der normalerweise außerhalb der Membran gesunder Zellen liegt, bedeutet für einen Organismus eine mehrschichtige Irritation, deren immunologische und übrige Reaktionen nicht zu übersehen sind. Lösliches CD4 könnte sich auch an MHC-II-Glycoproteine binden und deren normale Funktion beeinträchtigen, was beispielsweise bei AIDS-Kranken die Immunschwäche noch verschlimmern würde. Ferner müßte lösliches CD4 wiederholt in hoher Dosierung parenteral gegeben werden (Spektrum der Wissenschaft - Dez. 88, S. 116).

Als Ausweg aus diesem Problem wurde vorgeschlagen, kleine Abschnitte des CD4 herzustellen, Proteinbruchstücke, die so strukturiert wären, daß sie gerade noch von gp 120 erkannt würden. Gelänge dies, so bliebe immer noch die Frage, ob dieses Proteinbruckstück nicht immunologisch zu Gegenregulationen im vivo führen wird und heute noch unbekannte Störungen des sehr labilen Aidskranken auslösen würde.

1990 wurde von M. LANGE, (USA, s.a. Ärztliche Praxis 15, S. 28 ff. Werk-Verlag,D- 8032 Gräfelfing) deutlich gemacht, daß bei AIDS- und ARC-Patienten der Weg zum "Point of no Return" keineswegs nur eine Frage der Virusproduktion ist. Vermutlich setzt HIV als Antigen immunologische Pathomechanismen in Gang, die zur Eskalation neigen. Ähnlichkeiten mit Auto-Immunkrank-heiten (z.B. Lupus Erythematodes) zeigen, daß es nicht genügen kann, nur eine Anti-HIV-Chemotherapie zu betreiben. Es scheint vielmehr unabdingbar, diese autoimmunologischen Verselbständigungen (Perpetuierung) mit in die Therapie einzubeziehen, also eine neue Form der "antieskalativen Chemotherapie" zu konzipieren und zu entwickeln.

Nach LANGE kommt es im Rahmen einer HIV-Infektion zu einer extremen Komplement-Aktivierung, der Anaphylaxiefaktor C5 a induziert die Produktion von Tumor-Nekrosefaktor und Interleukin 1. HIV kann diese Kaskade zur Eskalation ankurbeln. Dies ist von mehreren Arbeitsgruppen nachgewiesen worden. Schon daraus läßt sich zwanglos ableiten, daß eine erfolgversprechende Anti-HIV-Chemotherapie sich nicht allein auf die HIV-Inhibition o.a. beschränken darf, sondern zumindest partiell derartige autoimmunologische Circulus vitiosus-Mechanismen unterbrechen muß.

Das individuelle, eskalative Zusammenwirken der diversen Noxen (HIV, Verursacher opportunistischer Infektionen, Autoimmun-Kaskaden u.w.) muß noch besser verstanden werden, um zu einer wirklich curativ nützlichen "Chemotherapie" zu kommen. Erst so können sich neue Therapieansätze ergeben.

Die Erfolgsbilanz der derzeitigen Therapien (z.B. mittlere Lebensdauer nach Infektion 13 - 18 Monate, 5-Jahres-Überlebensrate 3,4% (San Francisco 1981 - 87, 4000 Patienten, veröffentlicht in J. Am. Med. Ass. 263 (1990), 402) beweist, daß die derzeit ausgeübten rein chemotherapeutisch orientierten Methoden nicht zufriedenstellend sind.

Mach, W.J., der Erfinder, konnte schon 1961 gemeinsam mit WITTICH und STUHLFAUTH nachweisen, daß die von MACH dargestellten Naturfarbstoffe vom Neo-Penichromin-Typ in kleinen Mengen (parenteral) als "endogene Steuerstoffe " am Menschen wirken und z.B. die Nebennierenrinden-Aktivität steigern.

Dies ist vermutlich der erste Hinweis dafür, daß Redoxpigmente mit chinoiden Einheiten "anti-eskalative" Mechanismen des Menschen anzuregen vermögen, ohne selbst ein Protein bzw. Hormon zu sein und dies nicht auf dem Wege der unspezifischen Reizköperwirkung.

Eine zukünftige HIV-Chemotherapie muß also gleichzeitig (aus immunologischer Sicht) antieskalative Elemente enthalten, um den "Weg zum Point of no Return" zu hemmen.

Es ist Aufgabe der Erfindung, einen möglichst auch parenteral verabreichbaren Wirkstoff zu finden, der eine Neutralisation von Viren erreicht, ohne zytotoxische Effekte und ohne unerwünschte immunologische Reaktionen im Organismus des behandelten Patienten auszulösen.

Ferner ist es auch Aufgabe der Erfindung, einen Wirkstoff zu finden, der möglichst gegenüber auch veränderten/mutierten Virusformen aktiv ist.

Die Aufgabe wird erfindungsgemäß gelöst durch eine Wirkstoffgruppe zur restitutiven Chemotherapie bei viralen Infektionskrankheiten, erhältlich durch
I) Herstellung polysaccharidreicher Lignin-Einheiten (PLP) durch:
   Extraktion von Holz oder holzartigern materialien auch aus (oder) pflanzlichen Zellkultuen (A) in wässrigem Medium in schwach saurem bis alkalischem Milieu; btrennen der unlöslichen festen Bestandteile;
II) Herstellen niedermolekularer polysaccharidarmer lignoider Einheiten (LPL) durch:
   wässrig-alkalische Extraktion (pH 7 - 14) von Holz-Inkohlungsprodukten oder biokonvertierten holzartigen Materialien (B);
   Abtrennen der alkaliunlöslichen festen Bestandteile;
III) Herstellung eines wasserlöslichen Umsetzungsproduktes LD(PLP=LPL) mit niedrigem Molekulargewicht (LD) durch:
   Umsetzung des Produktes des Schrittes I, den polysaccharid-reichen Lignineinheiten (PLP) mit den Produkten des Schrit-tes II, den polysaccharidarmen lignoiden Einheiten (LPL) unter wässrigalkalischen Bedingungen (pH 9-12);
   Isolation der Low Dalton-Fraktionen dieses Umsetzungsprodukts durch Ultrafiltration bei nominellen Trennschnittwerten von etwa 15 bis 40 kD, wobei das Retentat verworfen wird;
   Behandlung der Lösung mit H⁺-Kationenaustauscher bei pH 3 bis 7,0, sowie Weiterverarbeitung der sauren Lösung der Wirkstoffgruppe LD(LPL=PLP).

Es kann bevorzugt sein, daß man in Schritt I) polysaccharid-reiche Lignin-Einheiten HD(PLP) mit weniger als 100 kD gewinnt, indem nach der Extraktion in wässrigen Medien und Abtrennen der unlöslichen festen Bestandteile eine Einstellung des pH-Wertes auf neutral bis schwach sauer erfolgt; eine Molekularfiltration, bevorzugt Ultrafiltration bei einem geeigneten Trennschnittwert von kleiner als 100 kD; bevorzugt kleiner als 70 kD; erfolgt und das Retentat verworfen wird;
die derart erhaltene Lösung von Substanzen mit niedrigem Dalton wird in Schritt III) weiterverarbeitet.

Bei einer bevorzugten Ausführungsform der Erfindung werden bei Schritt II niedermolekulare polysaccharidarme lignoide Einheiten LD-LPL mit weniger als etwa 40 kD hergestellt, indem nach der wässrig-alkalischen Extraktion und Abtrennen der alkaliunlöslichen festen Bestandteile eine alkalische Behandlung bei pH-Werten über 8 durchgeführt wird und danach eine Molekulartrennung durch Ultrafiltration bei nominellem Trennschnitt von 15 bis 35 kD erfolgt und das so erhaltene Produkt mit niedrigem Dalton in Schritt III weiterverarbeitet wird.

Es kann günstig sein, falls nach der Umsetzung der polysaccharidreichen Produkte des Schrittes I (PLP) mit den polysaccharidarmen Produkten des Schrittes II (LPL) eine Feststofftrennung durchgeführt wird und sodann die verbleibende Lösung weiterverarbeitet wird.

Die Weiterverarbeitung der aus Schritt III erhaltenen sauren LD(LPL=PLP)-Lösung kann durch Partikelfilterung und Pyrogenentfernung durch Filterung und anschließender Sterilisation sowie ggf. Konfektionierung erfolgen.

Vorteilhafterweise werden die zu extrahierenden holzartigen Materialien in Schritt I) ausgewählt aus der Gruppe bestehend aus:
A1: natives Holz,wie bspw. Kernholz von Bäumen in Form von "milled wood", Weichholz, Hartholz, PLP-Systeme, wie sie bspw. in Kernobst- oder Nußschalen auftreten, allgemein verholzte Pflanzenbestandteile, bevorzugt als entharzte Ausgangsprodukte; Gräser, wie Esparto, etc.
A2: Holz-Analoga, wie biotechnologisch darstellbaren holzigen Substanzen durch Aufbereitung (Züchtung) pflanzlicher Zellkulturen
A3: synthetische Holz-Analoga über Darstellung von FREUDENBERG-Dehydrierungspolymerisaten (DHP) von Mono- und/oder Dilignolen u. dgl. und Aufpfropfen der DHP's auf Polysaccharide;
A4: Lignin-Polysaccharid-Komplexe, (erhältlich durch alkalische Extraktion von Chlorit-Holzzellulose);
   sowie Mischungen (A1, A2, A3, A4) derselben.

Bevorzugt sind die in Schritt II) verwendeten pflanzlichen Ausgangsmaterialien ausgewählt aus der Gruppe bestehend aus:
B1: nativen Produkten der Inkohlung
B2: durch lignolytisch aktive Mikroorganismen, wie Weißfäulepilze, biokonvertiertes Holz
B3: durch Einwirkung isolierter lignolytischer Enzyme (wie Phenoloxidase) biokonvertiertem Holz,
   oder Mischungen (B1, B2, B3) derselben;

Vorteilhafterweise werden als Basen KOH, NaOH, LiOH und Ammoniak verwendet, es können aber auch andere Basen eingesetzt werden.

Bei einer typischen Ausführungsform der Erfindung liegen die polysaccharidreichen Lignin-Einheiten (PLP) nach Schritt I in einer Konzentration von 0,05 - 10 Gew%; bevorzugt etwa bis 2 Gew.% und ganz besonders bevorzugt in etwa 0,1 Gew.% vor.

Beispielsweise wird das Produkt des Schritts I, PLP bei einem pH-Wert von etwa 12 - 14, bevorzugt in KOH über 1 - 10 Tage bei Raumtemperatur (20 °C) extrahiert und die PLP-Extraktion bei erhöhter Temperatur bis zu 120 °C, wobei bei Temperaturen über etwa 100 °C im Autoklaven gearbeitet wird, durchgeführt.

Es kann auch bevorzugt sein, daß die Holz-Inkohlungsprodukte ausgewählt sind aus der Gruppe bestehend aus Ligniten, Farbkohlen, Braunkohle.

Die alkalische Extraktion mit 0.1-0,8 M KOH erfolgt bei einer bevorzugten Ausführungsform mit 0,4 M KOH, bei Raumtemperatur. Bspw. kann die alkalische Fragmentierung über 7 bis 10 Tage bei pH 11 (KOH) bei Raumtemperatur erfolgen.

Die Umsetzung in Schritt III zur Wirkstoffgruppe kann bspw.bei einer Temperatur zwischen Raumtemperatur und 120°C und bei einem pH-Wert von 8-14, bevorzugt 10-12, erfolgen.

Besonders bevorzugt wird das LD(PLP=LPL)-Umsetzungsprodukt bei Raumtemperatur einer Molkulartrennung zur Gewinnnung eine LD-Fraktion unterworfen, bevorzugt einer Niederdruck-Ultrafiltration an alkalifesten Ultrafiltrations-Membranen mit einem nominellen Trennschnitt zwischen 18 - 38 kD.
Ferner wird das LD (PLP=LPL) Umsetzungsprodukt einem Austausch der Alkaliionen durch Kunstharzkationenaustauscher in der Wasserstofform unterworfen, bis zu einem stabilen pH-Wert im Bereich von 4 bis 6,8, bevorzugt 5-6, wobei anschließend die saure LD-(PLP=LPL)-Fraktion durch Filterung von Pyrogenen befreit und anschließend bspw. durch Autoklavierung, bspw. bei 121 °C über 14 Minuten sterilisiert wird.

Die wäßrige Lösung der Wirkstoffgruppe (PLP=LPL) wird in an sich bekannter Weise, ggf. mit Hilfsstoffen, zu einer fließfähigen pharmazeutischen Zubereitung verarbeitet werden, je mach Anwendungsform, bspw. mit einer Salbengrundlage zu einer Salbe zur äußeren Anwendung oder mit einer Basislösung zu einer Lösung zur Injektion oder auch zur äußeren Anwendung.

Dabei kann die wäßrige Lösung der Wirkstoffgruppe LD(PLP=LPL) in an sich bekannter Weise zu Ampullen für Injektionszwecke verarbeitet werden. Bevorzugt kann die Wirkstoffgruppe zur Bekämpfung von Virus-Infektionen eingesetzt werden, bspw. als parenteral oder lokal anwendbares virenhemmendes Therapeutikum zur Bekämpfung viraler Infektionskrankheiten, wobei die Viren u.a. Retroviren, insbesondere vom HIV-Type (AIDS-Viren) sein können .

Es kann auch günstig sein, die erfindungsgemäße Wirkstoffgruppe in Form eines Chelats von Edelmetallen, wie Pt, Au, Pt einzusetzen.

Vorteilhafte Weiterbildungen der Erfindung ergeben sich aus den Unteransprüchen.

Jahrelange Tests an Probanden haben gezeigt, daß parenterale Tagesgaben von 2-7 Ampullen zu 2 mg Theapeutikum/24 Std. schwere Influenza-Infektionen (auch als viral-bakterielle Mischinfekte) innerhalb von 24 bis 48 Stunden ohne Nebenwirkungen zu cupieren vermögen. Selbst schwere Asthmapatienten, die an schweren grippalen Infekten litten, können durch 10 mg/80 kg Körpergewicht/24 Stunden ohne Einsatz von Kortikosteroid-Dosen über 5 mg so beeinflußt werden, daß der gefährliche status asthmaticus vermieden werden kann.

Beim experimentellen Bronchospasmus am Tier zeigt sich eine gesicherte Wirkung atoxischer Dosen (parenteral) im Sinne einer Hemmung des Bradikininspasmus (Serotoninhemmung und Hemmung der Prostaglandinsynthese)

Diese u.a. Wirkungen sind für eine Wirkstoffgruppe absolut ungewöhnlich und zeigen, daß dieses Wirkstoffsystem den Forderungen von LANGE, M. (Zitat s.o.) näher kommt als die bisherigen HIV-Hemmstoffe.

Besondere Vorteile der Erfindung sind unter anderem:
- in der HIV-infizierten Zellkultur (Human-Lymphozyten) (Test s.u.) kommt es im Bereich nicht zytotoxischer Konzentrationon zu einer signifikanten Hemmung der Syncytienbildung ("Anti-HIV-Aktivität")
- an Zellkultursystemen aus zentralen Neuronen (aus dem Hippocampus bzw. Cortex) kommt es unter streng definierten Testbedingungen sowohl an künstlich zusätzlich vorgeschädigten Neuronen und an nicht zusätzlich vorgeschädigten Neuronen zu einem signifikanten Zuwachs an Überlebensfähigkeit ("Survival-Effekt") was biologisch gleichbedeutend ist mit einer Inhibition eskalativer zellulärer Prozesse (flankierende restitutive Wirkung).
- Da Neuronen offensichtlich ein bevorzugtes Zielorgan für HIV, aber auch konventionelle Viren sind (in situ am Patienten) ist diese Eigenschaft von besonderer curativer Bedeutung.
- orientierende Tests aus dem Fachbereich der M.Parkinson-Grundlagen-Forschung zeigen, daß es u.a. sehr wahrscheinlich ist, daß die Wirkstoffgruppe zum Durchtritt durch die Blut-Hirn-Schranke befähigt ist.
- Auch bei Langzeitmedikation (s.c.) über Jahre tritt keine unerwünschte Sensibilisierung auf, es fehlt jede Allgemeinreaktion auf die Medikation wie reaktives Fieber u.ä., das Blutbild wird nicht medikationsabhängig in irgend einer Weise verschlechtert.

- Bei Infektionskrankheiten durch konventionelle Viren (Influenza, Windpocken u.s.w.) kommt es zu einer raschen und vollkommenen Restitution mit Entfieberung ohne unerwünschte Nebenwirkungen auf die Kreislauf-Leistung
- da es sich um einen non-protein-Wirkstoff handelt, wirkt er weder als Co-Antigen noch als fiebererzeugender Reizstoff.
- die parenterale Medikation ist einfach und problemlos (s.c.)
- Die curativen Dosen liegen bei ARC-Patienten bei 20 - 40 mg/Woche
- selbst bei extremster Überdosierung (z.B. 48 mg/24 Std, wiederholt) kommt es weder zu akuten noch zu chronischen Folgeschäden lokaler oder systemischer Natur.

Primäre Schwerpunkt-Indikationsgebiete für das neue Wirkstoffsystem sind - ohne allerdings eine Einschränkung auf diese Gebiete zu beabsichtigen:
- HIV-Infektionen
- ARC-Fälle mit opportunistischen Infektionen
- Zellschädigungen durch chronische Virusinfektionen allgemein
- curative Beeinflussung der fallweisen Multiresistenz von Krebszellen gegen konvenionelle Antitumor-Chemotherapeutika
- Krankheiten des Zentralnervensystems
Bevor die Erfindung näher erläutert wird, sollen zunächst die verwendeten Kurzbezeichnungen und Abkürzungen zum besseren Verständnis aufgeführt werden:
- LD =: Low - Dalton (niedermolekular, hier max. 3000 D)
- HD =: High-Dalton (höhermolekular, hier: > 3000 D)
- P =: Polysaccharidketten, nativ gebunden an Lignin-Einheiten bszw. mit Polyosen als Zwischenglieder zwischen Lignin und Cellulose.
- L =: (native) Lignin-units in allen natürlichen Varianten
- LD-L =: (Low-Dalton Lignin-.Einheiten - max 3000 D)
- HD-L =: (High-Dalton Lignin-Einheiten)
- (PLP) =: Zuckerreiche (Polysaccharide/Polyosen) Lignin-Einheiten, wie sie als milled-wood-Lignin bspw. aus Holzmehl mit Wasser bei alkalischem pH extrahierbar sind und ebenfalls in Varianten, gegeben durch die Holzbiosynthese, vorliegen).
- (LPL) =: Zuckerarme lignoide Einheiten, wie sie aus Xylem-Inkohlungsprodukten, wie Lignit, Farbkohlen (bis Steinkohlen) durch alkalische polare (wässrige) Medien u.a. extrahierbar sind, aber durch den Prozeß der Inkohlung stark verarmt an assimilierbaren Energieträgern vonm Typ Polysaccharide sind (Mikrobieller Abbau vor der Inkohlung u.ä.)
- LD(L-PL) =: Zuckerarme lignoide Einheiten, wie aus Inkohlungsprozessen, die durch alkalische wässrige Medien u.a. extrahierbar sind, aber durch den Prozess der Inkohlung stark verarmt an assimilierbaren Energieträgern vom Typ Polysaccharide sind, mit Low Dalton (wasserlölich auch bei pH unter 7,0.)
- LD(PLP) =: Low Dalton Fraktion von zuckerreichen Lignin-Einheiten (s.o.).
- HD(LDP=PLP): Umsetzungsprodukt aus LD-Einheiten aus zuckerreichen und LD-Einheiten aus zuckerarmen L-Einheiten,
- LD(PLP=LPL): Die Low-Dalton-Fraktion des o.g. Umsetzungsprodukts, die sich zur erfindungsgemäßen biologischen Anwendung eignet.

Das erfindungsgemäße Umsetzungsprodukt (LPL=PLP) entwickelt gemäß den durchgeführten Untersuchungen einerseits eine virenhemmende Wirkung und ist andererseits praktisch nebenwirkungsfrei.

Ein Vorteil der erfindungsgemäßen, Zuckerketten aufweisenden Wirkstoffgruppe ist, daß es sich um ein "non-protein" handelt, das die Immunabwehr gegen körperfremde Eiweißstoffe nicht irritiert und daher für eine Langzeitmedikation geeignet ist.

Es wird angenommen, daß das erfindungsgemäße Wirkstoffgruppe die Andockung oder Anheftung eines Virus, insbesondere hier eines HIV-Virus, an eine Wirtszelle verhindert. Es ist höchstwahrscheinlich eine Glycoproteinatrappe, die, da sie kein Protein ist, keine Immunreaktion im negativen Sinne auslöst - es wirkt sogar am sensibelsten Testsystem, einer isolierten Hirnzelle, als "Überlebensfaktor".

In curativen Dosen (0,1 mg/kg Körpergewicht Mensch) wirkt die Wirkstoffgruppe auch bei Dauergabe nicht sensibi-lisierend, nicht toxisch und hat keine Nebenwirkungen. Die erfindungsgemäße Wirkstoffgruppe verfügt über hydrophile Domänen, die eine gute Wasserlöslichkeit bedingen.

Zusammenfassend ist festzustellen, daß die erfindungsgemäße Wirkstoffgruppe auf Grund ihrer Nicht-Proteinstruktur und Nontoxizität zur Langzeitgabe geeignet ist.

Ein besonderer Vorteil der erfindungsgemäßen Wirkstoffgruppe ist, daß es sich auch zum Schutz von Nervenzellen im Sinne eines Überlebensfaktors eignet.

Ein ganz besonderer Vorteil der erfindungsgemäßen Wirkstoffgruppe ist seine Vielfalt, die dafür sorgt, daß stets verschiedenste Konformationen des Wirkstoffes und Ladungsver-teilungen des Wirkstoffes für den Virus angeboten werden.

Von vielen Viren, insbesondere den HIV-Viren, ist bekannt, daß diese sich äußerst rasch ändern und anpassen, sodaß eine einzige Konformation gegen einen mutierten Virus nicht mehr wirksam ist.

Die erfindungsgemäß eingesetzten Gluco-Lignin-Einheiten sind komplizierte Polysaccharid-Polyose-Lignin-Systeme, die aufgrund Ihrer sterischen und ladungsverteilungsmäßigen Vielfalt die verschiedensten Glucorezeptoren absättigen können. Die erfindungsgemäße Wirkstoffgruppe (non-protein) (niedermole-kular und wasserlöslich) besteht im wesentlichen aus an Zuckerketten (Polysaccharidketten) verarmten Phenylpropan-Einheiten, mit chinoiden Domänen (Ortho- bzw. Parachinoid).

Lignoide molekulare Domänen, die eine Affinität zu Zuckerseitenketten haben und teilweise in sterisch-spezifischer Weise an Glukomolekülen verarmt sind, findet man in der Natur in sog. Inkohlungsprodukten; also in Farbkohlen, Ligniten aber auch in Spuren in Steinkohlen, Im Zuge der über Jahrmillionen verlaufenden Inkohlung kam es zu einer Verarmung an Polysaccharid-ketten, bzw. Polyosen, die die biologische Verknüpfung zwischen den Lignin-Einheiten und der Cellulose bilden - dies kommt durch den mikrobiologischen Abbau, oxidative Langzeit-Umsetzungen etc. zustande, die zu einem Kohlenhydratverarmten Inkohlungsprodukt führen.

Ferner ist ein Vorteil der Erfindung, daß das Molekülsystem dazu befähigt, ist, Zellmembranen biologisch zu durchdringen, wie durch Messungen an Nervenzellen und an Leberzellen in situ experimentell nachgewiesen werden konnte.

Durch wissenschaftliche Untersuchungen konnte festgestellt werden, daß die Wirkung der Wirkstoffgruppe auf infizierte Zellkulturen überraschend signifikant ist und keine wesentlichen Nebenwirkungen toxischer Art eintreten.

Demzufolge ist die erfindungsgemäße Wirkstoffgruppe
für eine Langzeitmedikation geeignet; es tritt bei Langzeitgabe kein Wirkungsverlust auf; es kann auch im Verdachtsfall ohne Risiko eingesetzt werden und ist mit geeigneten Wirkstoffen unspezifischer Art kombinierbar. Allgemein zeigt es eine restitutive Wirkung und ist problemlos injizierbar.

Nachfolgend soll anhand von Ausfuhrungsbeispielen sowie der begleitenden Zeichnung die Erfindung näher erläutert werden. Dabei zeigt:
- Fig. 1a:: Ein Herstellungsschema für eine erfindungsgemäße Wirkstoffgruppe
- Fig. 1b:: Ein weiteres Herstellungsschema für eine erfindungsgemäße Wirkstoffgruppe
- Fig. 1c:: Eine weitere Variante der Herstellung einer erfindungsgemäßen Wirkstoffgruppe
- Fig. 2a - 2e:: Fluoreszenzspektren für Lösungen der erfindungsgemäßen Wirkstoffgruppe bzw. ihrer Vorläufer gem. Beispiel 1 in Wasser

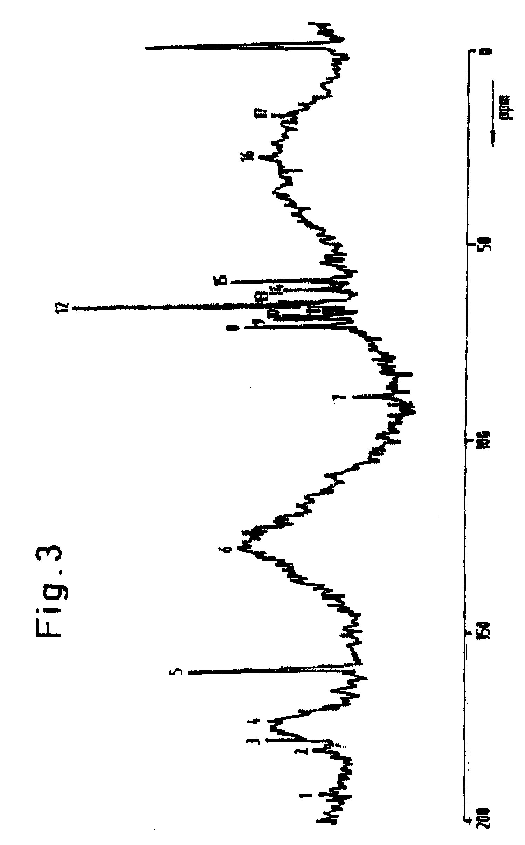

- Fig. 3:: Ein C13-Spektrum einer erfindungsgemäßen Wirkstoffgruppe.
- Fig. 4: graphische Darstellung der LDH-Aktivität in Ratten-Cortex-Kulturen unter Glucosemangel

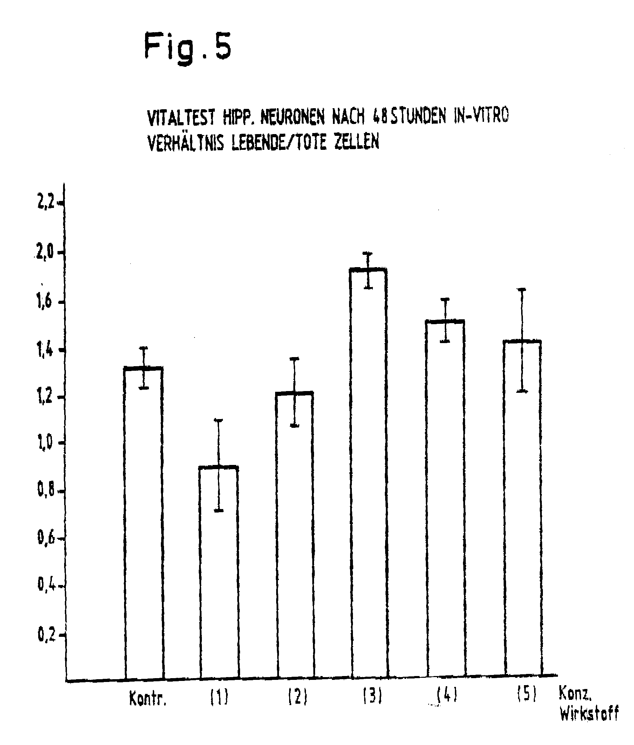

- Fig. 5: graphische Darstellung des Vitaltests mit Hippocampus-Neuronen nach 48 Std. in-vitro
- Fig. 6: Aufzeichnung der Extinktionsmessung in Abhängigkeit von der Bestrahlungsdauer der Wirkstofflösung

### Beispiel 1

### Herstellung einer erfindungsgemäßen Wirkstoffgruppe

### Schritt I.

### Herstellung der zuckerreichen Lignin-Fraktion (PLP)

300 g gemahlenes Kernholz der sibirische Lärche (Larix sibirfiaca) werden als milled wood in 6000 ml entmineralisiertem Wasser durch Rühren dispergiert und 150 g KOH zugegeben. Dieser Ansatz wird unter Rühren 3 Tage bei 46 bis 66°C gehalten (dielektrische, intermittierende Erhitzung). Anschließend werden die Feststoffe über eine Nutsche abgetrennt, der Rückstand verworfen, das Filtrat blank zentrifugiert und durch portionsweise Zugabe eines hochsauren Kunstharz-Ionenaustauschers (Amberlite IR 120) in der Wasserstoff-Form, der frisch reaktiviert worden war, unter ständiger pH-Kontrolle über eine geeichte Glaselektrode langsam auf pH 11,5 eingestellt. Der Ionenaustauscher wird abgetrennt und verworfen. Die blanke deutlich goldgelbe Lösung wird durch Molekulartrennung mittels Ultrafiltration an alkalifesten Ultrafiltrationsmembranen mit einem nominellen Trennschnitt von 100 000 Dalton von pyrogenen Stoffen und Partikeln befreit. Von dieser blanken gelben Matrix-Lösung wird nun eine Probe von 20 µl entnommen, in einer Quarzküvette (1cm) mit 3 ml Wasser für Spektralzwecke verdünnt und gemischt und eine Serie von Fluoreszenzanregungsspektren bei folgenden Wellenlägen registriert: 450 nm, 490 nm 520 nm.

Das Fluorimeter wird hinsichtlich der Empfindlichkeit so eingestellt, daß die relative Fluoreszenz-Emission bei der Wellenlänge 450 nm bei 100 oder in der Nähe davon liegt. Dann wird das Anregungsspektrum bei 450 nm aufgenommen (Schreiber 2cm/Min., Anregungswellenlängenvorschubsgeschwindigkeit 100 nm/min, Gerät: KONTRON (SCHWEIZ),Typ SFM-23 aufgenommen (registriert).
Zur Kontrolle wird auch das Anregungsspektrum bei 490 nm aufgenommen und ebenfalls bei 520 nm. Es wurde eine gelbe Lösung der PLP-Matrix erhalten.

### Schritt II:

### Herstellung der zuckerarmen Lignoid-Fraktion (LPL)

600 g eines "milled" (feinvermahlenen) Lignits werden bei Raumtemperatur (20 °C in 10 000 ml einer 1,68 Gew-%igen KOH-Lösung unter Rühren dispergiert; teilweise gelöst und nach 9 Stunden in einer Schnellzentrifuge von Feststoffen getrennt und das Sediment verworfen. Nach 24 Std. wird eine weitere Feststofftrennung in der Schnellzentrifuge durchgeführt und der Feststoff verworfen. Nach einer alkalischen Fragmentierung über 5 Tage bei 20°C wird nochmals das bisher gewonnene Zentrifugat einer Feststofftrennung unterworfen und nunmehr durch eine Ultrafiltrationsanlage mit alkalifesten Ultrafiltrationsmembranen mit einem nominellen Trennschnitt von 30 000 Dalton einer Molekulartrennung unterworfen. Die Ultrafiltration wird nach Vorliegen von 6,7 Litern Filtrat abgebrochen. Das Ultrafiltrat wird mit einem frisch aktivierten hochsauren Ionenaustauscher (Amberlite IR 120) unter Kontrolle einer geeichten Glaselektrode auf pH 5,5 eingestellt.

Diese schwach saure Lösung wird sofort an einem Ultrafilter zur Abscheidung von Pyrogenen filtriert. Anschließend erfolgt eine thermische Sterilisation im Autoklaven bei 121 °C über 15 Minuten. Zur Bestimmung der Feststoffkonzentration werden Proben gezogen und bei einer Temperatur von 85 °C bis zur Gewichtskonstanz eingeengt (Infrarotstrahler). Das so gewonnene Ultrafiltrat wird nach Einstellung auf die gewünschte Konzentration als LPL-Anteil bzw. Zuckerarme Lignoid-Fraktion (LPL) zur Reaktion mit dem Produkt des Schrittes I verwendet (s.o.).
Nachfolgend wird das C13-Spektrum der eingesetzten LPL-Fraktion (Produkt des Schrittes 2) diskutiert:

### 13C-NMR-Spektren

Aufgrund des 13C-NMR-Spektrums unterscheidet sich diese Probe vor allem durch ihre hohe Anzahl von Carboxylgruppen vom natürlichen Lignin, die durch ein mittleres Multiplett bei etwa 168-180 ppm angezeigt werden. Dabei sollten die Carboxyl-C-Atome aufgrund fehlender Kern-Overhauser-Effekte und Spin-Spin-Wechselwirkungen mit direkt gebundenen Wasserstoffkernen noch besonders schwache Signale ergeben, so daß ihre Konzentration noch höher liegen müßte als die relative Intensität ihrer Signale. Entsprechendes gilt auch für die Alpha-Carbonyl-C-Atome, die im Bereich 190,5-198 absorbieren, jedoch wesentlich schwächere Intensitäten zeigen. Das scharfe Singulett bei 158,2 ppm läßt sich am besten dem Kohlenstoffatom 4 in p-Hydroxyphenylgruppen zuordnen (zur Bezeichnung der C-Atome vgl. H.D. Lüdemann und H. Nimz, Makromol. Chemie, 175, 2409 (1974). Da aber die Messungen von entsprechenden Modellsubstanzen in Hexadeuteroaceton als Lösungsmittel und mit einem internen Standard vorgenommen wurden H.D. Lüdemann und H. Nimz, Makromol. Chemie, 175, 2393 (1974), sollten eventuell auch die wahrscheinlicheren C-Atome 3 und 5 in Syringylresten für dieses Signal in Betracht gezogen werden.

Das breite Multiplett (7) im Bereich bei 93-145 ppm muß den Kohlenstoffatomen 1,2,5 und 6 in Guajacyl- sowie 1,2,4 und 6 in Syringylresten zugeordnet werden. Die schlechte Auflösung des Spektrums in diesem Bereich deutet auf starke Kondensationen im Lignin hin, die sich aus seiner Vorbehandlung ergeben.

Im Bereich der aliphatischen Kohlenstoffatome lassen sich die Signale (8), (9) und (15) als typische Ligninsignale Arylglycerin-β-arylätherbindungen zuordnen, die den wichtigsten Bindungstyp in allen Ligninen darstellen. Eine Unsicherheit für die Zuordnung ergibt sich jedoch auch hier aus dem Lösungsmittel und dem externen Standard. Das relativ schwache Methoxylsignal bei 58,6 ppm deutet auf eine teilweise Entmethoxylierung während der Vorbehandlung der Probe hin. Die in diesem Bereich ebenfalls auftretenden gut strukturierten Signale (10), (11) und (12) finden sich nicht in typischen Ligninspektren (H.D. Lüdemann und H. Nimz, Makromol. Chemie, 175, 2409 (1974)) und deuten auf kondensierte Kohlenhydratanteile hin.

Schwer zu erklären ist auch das breite Multiplett (17) im Bereich bei etwa 0-55 ppm. Hier sollten vorzugsweise aliphatische Kohlenstoffatome absorbieren, die nicht direkt an Sauerstoff gebunden sind. Im Lignin kommen jedoch nur sehr wenige Atome dieser Art vor, z. B. die β-Kohlenstoffatome in Pinoresinoleinheiten und Alpha-Kohlenstoffatome in Dibenzyl-tetrahydrofuraneinheiten (Literatur: 1. H.-D. Lüdemann und H. Nimz, Makromol. Chem. 175, 2393, 1974; H.-D. Lüdemann und H. Nimz, Makromol. Chem. 175, 2409, 1974). Es müssen daher während der oxidativen oder anderen Vorbehandlung des Lignins derartige Kohlenstoffatome (z. B. in CO-CH₃,-CHOH-CH₃ oder -CH₂-C) aus dem Lignin entstanden sein, oder bei der Ligninpräparierung aus dem Holz übernommen worden sein.

Zusammenfassend läßt sich sagen, daß es sich bei der vorliegenden Probe um ein strukturell stark verändertes Lignin handelt, dessen Lignincharakter nicht mehr eindeutig nachzuweisen ist. Die Zuordnung der Signale wird erschwert durch die Verwendung von D₂O als Lösungsmittel, da es in Hexadeuteroaceton, in dem die Vergleichslignine gemessen wurden (H.D. Lüdemann und H. Nimz, Makromol. Chemie, 175, 2409 (1974)), nicht löslich ist. Ferner kann auch die Verwendung eines externen anstelle des internen Standards die chemischen Verschiebungen geringfügig beeinflussen, wodurch Unterschiede in den chemischen Verschiebungen von etwa 2 ppm durchaus auftreten können.
¹³C-Chemische Verschiebungen in ppm von TMS als externem Standard, aufgenommen in D₂O mit einem Varian XL-100-15-Spektrometer.

| Nr. | Delta(ppm) | Intensität | Zuordnung *) |
|---|---|---|---|
| (1) | 190,5-198 | schwaches Multiplett | Alpha-Carbonyl- und Zimtaldehydgruppen |
| (2) | 168-180 | mittleres Multiplett | Carboxylgruppen |
| (3) | 164,0 | schwach | |
| (4) | 158,2 | starkes Singulett | C-4 in p-Hydroxyphenyl (C-3/5 in Syringyl) |
| (5) | 150,5 | schwach | C-4 in Guajacyl |
| (6) | 148,5 | schwach | C-3 in Guajacyl |
| (7) | 93-145 | breites Multiplett | C-1/2/5/6 in Guajacyl und C-1/2/4/6 in Syringyl |
| (8) | 88,4 | schwach | C-β in Arylglycerin-β-arylethern |
| (9) | 70,0 | mittel | C-Alpha in Arylglycerin-β-aryläthern |
| (10) | 67,9 | mittel | |
| (11) | 67,4 | mittel | |
| (12) | 66,2 | schwach | |
| (13) | 64,8 | stark | C-Gamma in Phenylcumaranen und C-Gamma und C-β in β-1-Dilignoleinheiten |
| (14) | 63,8 | mittel | C-Gamma in Zimtalkoho len und Arylglycerin-β-arylethern mit Alpha-Carbonyl |
| (15) | 60,7 | mittel | C-Gamma in Arylglycerin-β-arylethern |
| (16) | 58,6 | mittel | OCH₃ |
| (17) | 0-55 | breites Multiplett | aliphatische, bevorzugt nicht an Sauerstoff gebundene C-Atome |

| | | | |
|---|---|---|---|
| *) vgl. hierzu: H.-D. Lüdemann und H. Nimz, Makromol. Chem. 175, 2409 (1974) | | | |

### Weiterverarbeitung in Schritt III:

Durch stufenweise Zugabe der zuckerarmen Lignoid-Fraktion (LPL) in definierter Konzentration von 2 mg/ml wird in Stufen von jeweils 5 µl Zugabe in die 3 ml Quarzküvette ("Fluoreszenztitration") das 520 nm-Anregungsspektrum aufgenommen, bis durch entsprechende Erhöhung der Zugabe ein signifikanter Emissions-Peak bei 465 nm auftritt und die Emission über den gesamten Emissionswellenlängenbereich stark ansteigt und bei der Emission 394-396 nm um die Größenordnung von 80% relative Emission ansteigt. Die zu dieser Fluoresnzenzsteigerung durch den Zuckerarme Lignoid-Fraktion (LPL)-Zustz notwendige Menge Zuckerarme Lignoid-Fraktion (LPL) wird bestimmt (hier waren es bspw. 10 µl der 0,2%-igen Lösung) und mit dem Faktor 2,5 multipliziert.

Die so bestimmte Zusatz-Menge von zuckerarme Lignoid-Fraktion (LPL)-Lösung zur Matrix-Lösung wird sodann mit der zur Verfügung stehenden Menge an Matrixlösung verwirklicht: z.B. zu 4000 ml Matrixlösung dieses Ansatzes werden gemäß der Anweisung also 5000 ml der 0,2%-igen Zuckerarmen Lignoid-Fraktion (LPL)-Lösung gegeben (120 µl Matrix benötigten 10 µl 0,2%-ige Zuckerarme Lignoid-Fraktion (LPL)-Lösung, also nach Multiplikation mit dem Faktor 2,5 ergeben sich auf 4000 ml Matrix 2000 mal 2,5). Bei 45 bis 65 °C. wird intensiv gemischt und nach 4 Stunden Reaktion bei 45 bis 65 °C eine Moleku artrennung an einer Ultrafiltrationsanlage mit alkalifester Ultrafiltrationsmembran mit nominellem Trennschnitt von 30 KD unterworfen, das Retentat verworfen und das Filtrat weiter verarbeitet. Mittels eines hochsauren Kationenaustauschers in H⁺-Form wird das Filtrat auf pH 5,5 eingestellt und nach Filterung zur Befreiung von Pyrogenen sofort thermisch sterilisiert.

Der Charge werden Proben für biologische und analytische Zwecke entnommen und nach Prüfung auf Identität durch Fluoreszenzspektroskopie und Pyrogenfreiheit zur Ampullierung freigegeben. Als Lösungsmittel für Ampullen zur parentheralen Anwendung dient 0,9%-ige NaCl-Lösung steril für Injektionszwecke.

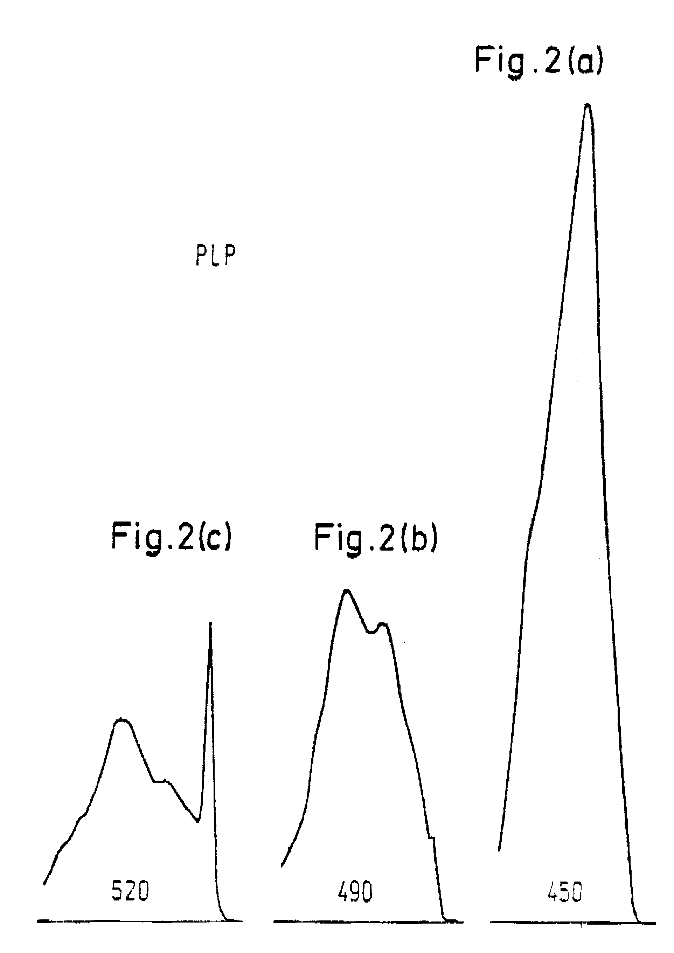

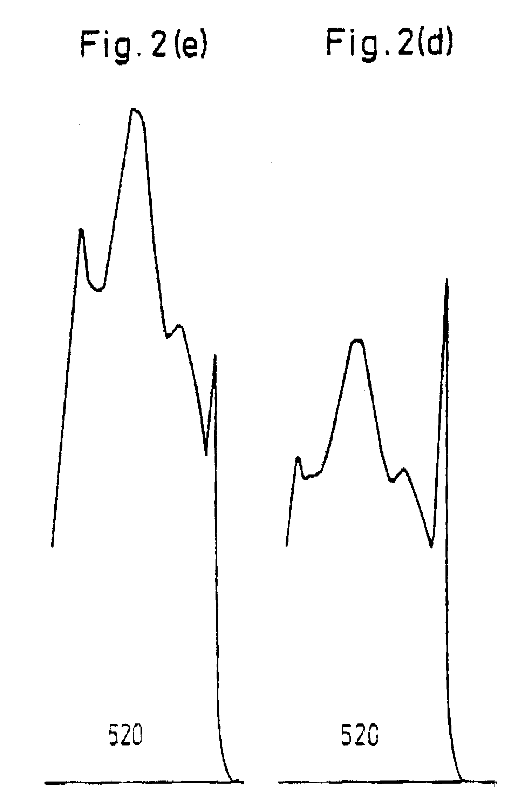

In Fig. 2a bis 2c sind zunächst die Fluoreszenzanregungsspektren bei 450 nm, 490nm und 520 nm Anregungswellenlänge der PLP-Lösung (Stoffgruppe A) gezeigt; die Fig. 2d und 2e zeigen nach dem Prinzip der Fluoreszenzanregungs-Titration bei 520 nm Anregungswellenlänge den meßbaren Einfluß der definierten Zugabe (hier zweistufig) von PLP in 5 µl-Stufen: Entscheidend ist hier das Auftreten und die Höhe des Emissionspeaks bei 466 nm Anregungswellenlänge.

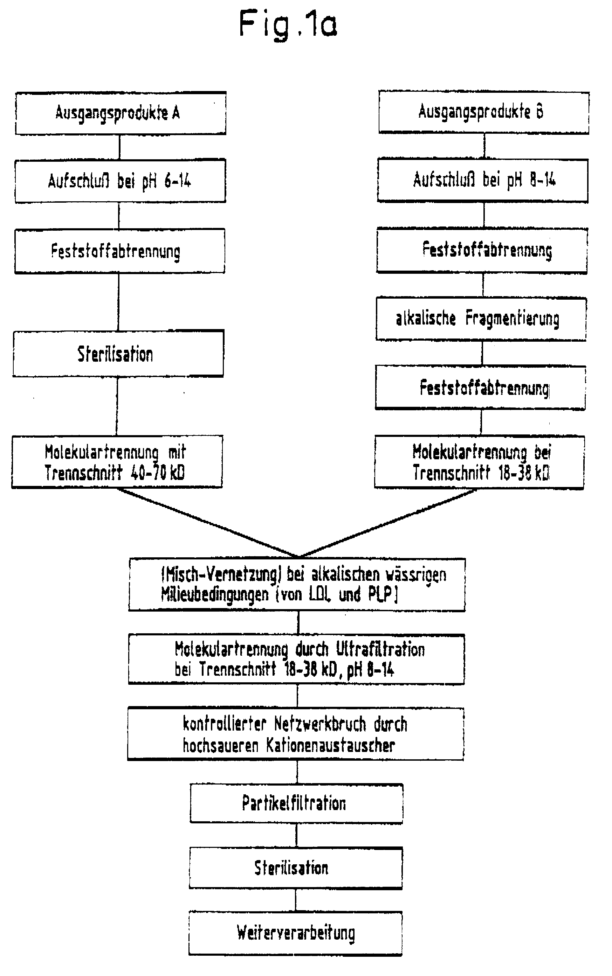

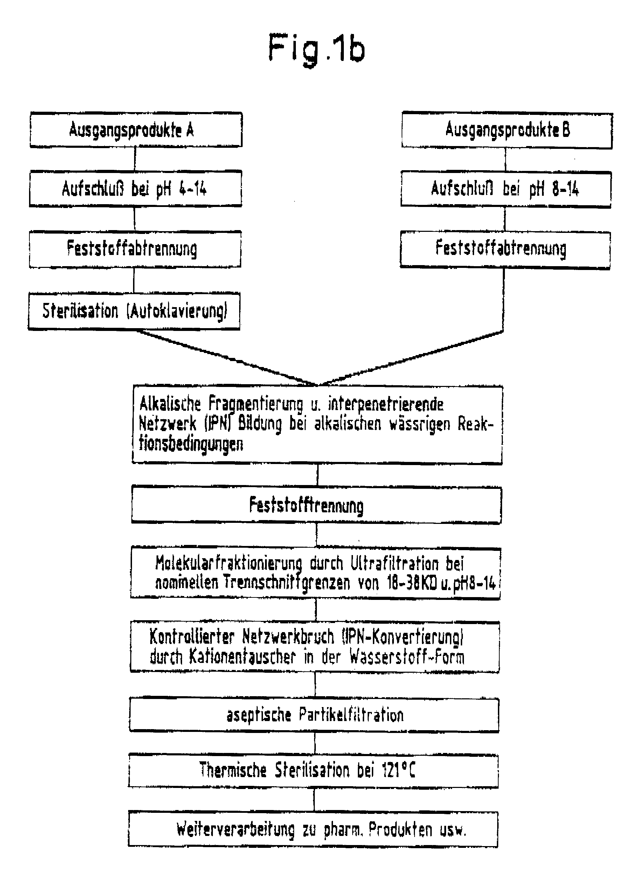

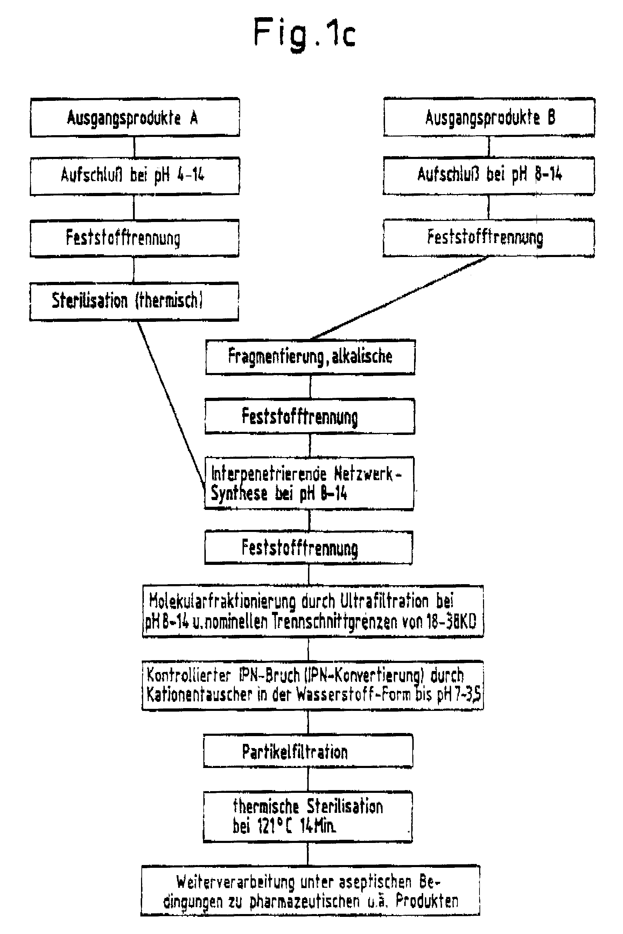

Das Reaktionsschema ist zur Verdeutlichung nochmals in Fig. 1 a gezeigt.
In den Fig. 1b und 1c sind mögliche Abwandlung dieses Verfahrens angegeben, wie sie ggf. einsetzbar sind.

### Beispiel 2

### Herstellung einer erfindungsgemäßen Wirkstoffgruppe

### Herstellung der zuckerreichen Lignin-Fraktion (PLP)

180 g gemahlenes Kernholz der sibirische Lärche (Larix sibirfiaca, zerkleinert auf eine Teilchengröße von 0,05 bis 0,315 mm in einer Schlagkreuzmühle, die vor der Extraktion in wässrigem Medium durch Extraktion mit Ethanol/Benzol und Ethanol von Extraktstoffen befreit wurden (TAPP-Standard T-12m, 1959) und 120 g Rotbuchenholz (Fagus sylvatica L), wie oben aufbereitet wurden gemischt und analog Beispiel 1 verarbeitet. Während Larix sibirifiaca im Kernholz Arabogalaktan (4 Teile Galaktose : 1 Teil Arabinose) enthält, liegen bei der Buche Xylanketten, besetzt mit Oxymethylglucuronsäure und L-Arabinose, vor, wodurch die Eigenschaften des Endproduktes modifiziert sind.

Die milled-wood Modifikation wird, wie in Beispiel 1, verarbeitet, wobei aber die Extraktion nach BECKMANN und LISCHE, Angewandte Chemie 34, 285 (1921) durchgeführt wird, indem mit 1M KOH im Autoklaven bei 121°C 3,5 Std.extrahiert wird.

### Beispiel 3

### Herstellung einer erfindungsgemäßen Wirkstoffgruppe

### (I) Herstellung der zuckerreichen Lignin-Fraktion (PLP)

Die Herstellung des PLP erfolgte wie in Beispiel 2.

### II) Herstellung der zuckerarmen Lignoid-Fraktion (LPL)

LPL wurde aus gepulverter eozäner Braunkohle, anstelle des Lignits, wie in Beispiel 1 angegeben, hergestellt. Im übrigen verlief die Herstellung, wie in Beispiel 1 angegeben.

### Beispiel 4

### Herstellung einer erfindungsgemäßen Wirkstoffgruppe

### I) Herstellung der zuckerreichen Lignin-Fraktion (PLP)

210 g Kernholz der Lärche, 60 g Kernholz der Buche und 30 g Fruchtschalenholz von prunus avium wurden, wie in Beispiel 2 angegeben, zu PLP verarbeitet.

### II) Herstellung der zuckerarmen Lignoid-Fraktion (LPL)

Miozäne Braunkohle, die in üblicher Weise durch Soxhlet-Extraktion mit Benzol von Harzen, Fetten und Wachsen gereinigt war, wurde wie in Beispiel 1 weiterverarbeitet.

### Beispiel 5

### Herstellung eines erfindungsgemäßen Wirkstoffgruppe

### I) Herstellung der zuckerreichen Lignin-Fraktion (PLP)

210 g Kernholz der Lärche, 90 g Synthese-Holz nach Freudenberg (Freudenberg K. Harkin JM, 1960, Modelle für die Bindung des Lignins an die Kohlenhydrate, Chem. Ber. 93, 2814-2819) wurden, wie in Beispiel 2 angegeben, zu PLP verarbeitet. Die übrige Weiterverarbeitung erfolgte wie in Beispiel 1.

### Beispiel 6

### Herstellung einer erfindungsgemäßen Wirkstoffgruppe

Ein alkalisch gewonnener Extrakt (A5) aus Chlorit-Holozellulose, gewonnen aus Buchenholzmehl (Fagus sylvatica L.), delignifiziert mit NaClO₂ (nach Feckl, 1981, Diss. Universität München) und mit 4%-iger KOH-Lösung nach FENGEL (1976, Holzforschung 30, S. 73 - 78) alkalisch extrahiert, wurde einer Feststofftrennung unterworfen, sterilisiert und am Beginn des dritten Tages in einer Menge von 900 ml (Konzentration 0,12 % Feststoffgehalt, Restligningehalt 2,3%) in die LPL-Lösung aus Lignit (hergestellt wie Beispiel 1) eingespeist. Das Volumen der 2,1%-igen Lösung (pH 11,4) betrug 9100 ml. Die Fragmentierungszeit betrug 9 Tage. Die Lösung wurde anschließend, wie in Fig. 1 b dargestellt, weiterverarbeitet, indem anschließend eine Feststofftrennung durchgeführt wurde und die verbleibende Lösung einer Molekularfiltration durch Ultrafiltration an alkalifesten Membranen mit nominellen Trennschnittgrenzen von 18-38 kD und einem pH-Wert von 8-14 unterworfen wurde. Anschließend wurde die Lösung durch H⁺-Ionenaustauscher angesäuert und einer aseptischen Partikelfiltration unterworfen. Das erhaltene Filtrat wurde thermisch bei 121°C im Autoklaven sterilisiert und konnte dann, wie in Beispiel 1 angegeben, weiterverarbeitet werden.

### Beispiel 7

### Herstellung einer erfindungsgemäßen Wirkstoffgruppe

Schritt I:
100 g gemahlenes (wie "milled wood") Cortex Cinnamoni wird 6 Tage mit 1000 g einer Lösung von KOH in demineralisiertem Wasser bei 60°C extrahiert (pH-Einstellung auf 8-9 mit KOH). Dabei wurde durch Sauerstoff-Einleitung in die Lösung eine Um-wälzung des Materials aufrechterhalten. Anschließend wurden die Feststoffe durch Zentrifugation abgetrennt und verworfen. Die derart erhaltene PLP-Lösung wurde thermisch im Autoklaven sterilisiert und sogleich gemeinsam mit dem LPL-Produkt des Schrittes II des Beispiels 1 in alkalischem Medium in der Wärme umgesetzt, wobei die weitere Verarbeitung wie in Beispiel 1 angegeben, erfolgte.

Diese Präparation ist in Fig. 1c nochmals verdeutlicht.

### Beispiel 8

600 g milled Farbkohle (eozän), identisch mit dem Produkt aus dem Beispiel 3, wird bei Raumtemperatur in 10 l einer Lösung von 110 g KOH und 60g NaOH in demineralisiertem Wasser über 2 bis 5 Stunden unter Rühren alkalisch aufgeschlossen. Vor der Auflösung der Hydroxide wird 20 g Aktivkohle in feinster me-chanischer Verteilung dem Wasser zugesetzt. Dieser Aktivkohle-anteil wird dann gemeinsam mit den Sedimentanteilen bei der nachfolgenden Vorzentrifugation abgeschieden und bedeutet so schon einen vorbereitenden Reinigungsschritt des für die alkalische Fragmentierung u.s.w. vorgesehenen Produktes.

Die Vorzentrifugation in Großraumzentrifugen, notfalls auch Becherzentrifugen, bspw. bei 4000 bis 5000 U/min ist erforderlich, da die Feststoffabscheidung (nächster Verfahrensschritt) bei maximaler schwerkraftwirkung durch Ultrazentrifugen (auch Schnellzentrifugen mit senkrechten Abscheidezylindern und ca 40 000 U/min) dann sofort zumn Erliegen kommt, wenn die Menge des Sediments den Wirkungrad im Klärzylinder senkt, wodurch jede industrielle Produktion unmöglich würde. Das Vorzentrifugat wird gesammelt, mit 8g Aktivkohle pro 10 l versetzt und umgehend einer Ultrazentrifugation (N.s.o.) so lange unterzogen, notfalls im Kreislauf, bis der Klärzylinder o.ä. der Ultrazentrifuge praktisch blank bleibt und nur noch minimalste Spuren eines lackartigen Sediments erkennen läßt.

Dieses nunmehr intensiv vorgereinigte Produkt (alkalische Wirkstoffgruppenrohlösung) wird nun beim Raumtemperatur gelagert und der "alkalischen Autofragmentierung" überlassen. Diese Fragmentierungsvorgänge werden durch laufende pH-Messungen verfolgt. Probeweise sollten ferner mindestens zweimal eine weitere Ultrazentrifugation wie oben durchgeführt werden, um die sich bildenden Sedimentation fähigen , aber aus pharmakolo-gischer Sicht unterwünschten Fragmentkondensate ohne echte Löslichkeit und molekulare Reaktivität laufensd auszuscheiden.

Nach der alkalischen Autofragmentierung, also nach etwa 9 Tagen, wird nochmals eine Feinstklärung mittels Ultrazentrifugation durchgeführt und die Lösung (immer noch hoch alkalisch) durch Ultrafiltration bei einem nominellen Molekulartrennschnitt von 30 000 Dalton einer schonenden Fraktionierung unterworfen und zwar in der Weise, daß bei einem Produktionsansatz (wie hier beispielsweise beschrieben) von 10 Litern die Ultrafiltration beendet wird, wenn ca 74000 ml Ultrafiltrat gewonnen wurden und das Retentatvolumen dann also ca 2000 ml beträgt.

Das Retentat wird verworfen, die Ultrafiltrationsanlage gründlich alkalisch gespült und das Filtrat dem nächsten Aufbereitungsschritt unterzogen. Der nun folgende Kontakt der Alkalisalze des Ultrafiltrates mit dem hochsauren Kunstharzkationenaustauscher (in der extremem Wasserstoff-Form) auch ein Vorgang, der für das Endproduktes von Bedeutung ist.

Die "Ansäuerung" des alkalischen Ultrafiltrats z. B. durch Zugabe von Säure, ja sogar die Anwendung von Ionenaustauscher-Säulen ist völlig ungeeignet, weil z.B. im letzteren Falle die Säule sich leicht zusetzt und dann ein völlig unbrauchbares Filtrat gewonnen wird.

Erfindungsgemäß muß der Kationenaustausch (Austausch der Ionen Na+ und K+ gegen H+) im Batchverfahren in einem Reaktionsgefäß durchgeführt werden und der Entionisierungsvorgang laufend durch eine geeichte Glaselektrode elektronisch gemessen werden.

Zielpunkt ist ein pH-Wert von 5.1 und nach Erreichen dieses Wertes muß die Zugabe des Ionentauchers sofort unterbrochen werden. Erfindungsgemäß sollte die Entionisierung unter ständiger Umwälzung des noch teilweise alkalischen Produktes durchgeführt werden und durch Turbulenzbildung, bsw. durch eine regelbare Umwälzmpumpe dafür gesorgt werden muß, daß nicht Bereiche mit hoher Ionentauscher Konzentration gebildet werden.

Da die Wirkstoffgruppe derzeit noch nicht befriedigend gemessen werden kann, ist die er-findungsgemäße Befolgung auch der kleinsten, experimentell gefundenen Reaktionsschritte nach wie vor die sicherste Methode, um zu pharmazeutisch absolut identischen erfin-dungsgemäßen Wirkstoffgruppeen zu gelangen.

Die nunmehr dem Kationenaustausch unterworfene Wirkstofflösung wird nun in bekannter Weise durch ein steriles Filter von Partikeln befreit, die während des Kontaktes mit dem ionenaustausch dort hineingekommen sein können, und sofort anschließend durch Autoklavierung (z.B. 121°C über 20 Minuten) sterilisiert. Dies Wirkstofflösung kann bei plus 4 bis 20°C 18 Monate absolut sicher gelagert werden und z.B zur Herstellung von Ampullenlösungen (Lösemittel z.B. 0.9%-ige Kochsalzlösung) verwendet werden.

Der hier durchgeführte Aktivkohleeinsatz ist keine unspezifische Reinigungsoperation, sondern es handelt sich hier um eine Beeinflussung der Reaktionssituation im Zuge der gezielten Fragmentierung: Grundsätzlich sollte mit Aktivkohle vom Typ "Pulver-Aktivkohle p.a." gearbeitet werden (Beispiele für Kenndaten derartiger Aktivkohlen: pH-Wert einer 5%-igen Lösung in Wasser, 20° Filtrat) 4.0 - 7.0; Methylenblauadsorptionsfähigkeit: 0.15%-ige Lösung: größer als 12 ml/0,1g).

Wesentlich ist die vielfältige Funktion der Aktivkohle z.B. durch ihren Gehalt an Oberflächenoxiden, die der Aktivkohle saure oder basische Eigenschaften verleihen können. Zwar sind reine kohlenstoffoberflächen wie beim Graphit hydrophob, die Oberflächenoxide bilden jedoch in situ hydrophile Bereiche, sodaß die Aktivkohle durch Wasser benetzbar wird und so Einfluß auf die Reaktionssituation im Zuge der Fragmentierung nimmt. Wichtig ist ferner die Korngrößenverteilung der eingesetzten Aktivkohler: etwa 50% sollten Partikel umfassen, die kleiner als 40 Mikron sind. Bei dieser Korngrößenverteilung lassen sich die Aktivkohlezusätze ohne Einsatz von Filterhilfsmitteln technologisch sauber abtrennen.

### Beispiel 9

Das in Beispiel 8 dargestellte, im "Ein-oder-mehr-Ionenprinzip" bereits mehrfach durch Ultrazentrifugation von Sedimentteilchen befreite Produkt wird mittels Mikrowellenerhitzung jeweils kurz auf 46°C erhitzt und dann wieder der Abkühlung bis auf Raumtemperatur überlassen. Das alkalische Produktgemisch wird nach einer letzten Feinstreinigung mittels Ultrazentrifugation (näheres s. Beispiel 8) genauso weiter verarbeitet, wie das Produkt gewonnen , nach Beispiel 8).

Durch diese erfindungsgemäß wiederholte physikalische erzwungene Anderung der Prozesse der Fragmentierung wird die Wirkstoffgruppe modifiziert.

### Beispiel 10

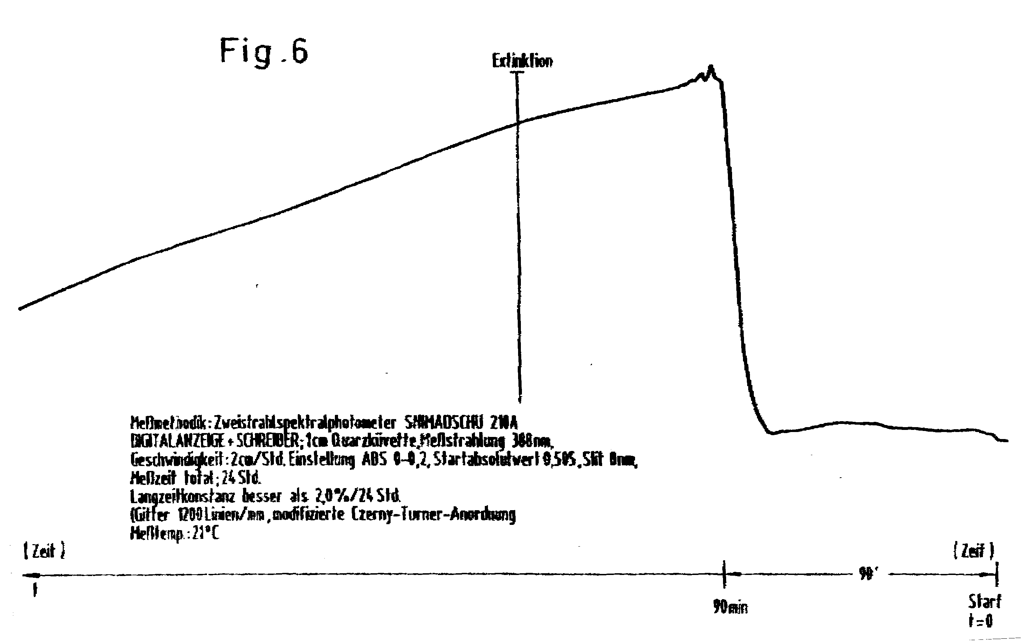

Reaktionsphotometrische Untersuchungen haben gezeigt, daß Therapeutika im Sinne der Erfindung z.B. nach Bsp. 8 hergestellt, durch Photonenaktivierung bei 388 - 378 nm intensiv zu modifizieren sind. Bei dieser Stoff-Umwandlung im Bereich der Photonenfänger-units der Wirkstoffgruppe kommt es zu Vorgängen im Sinne der Photochromie: Darunter versteht man reversible photochemische Reaktionen, bei denen der Stoff A in eine andere Form (Konformation) oder Verbindung B übergeht und diese Hin- und (oder Rückreaktion durch Absorption von UV- oder sichtbarem Licht ausgelöst wird. Die Therapeutika werden erfindungsgemäß solange mit der Wellenlänge 388 nm beinflußt, bis es unter Kontrolle der Extinktion z.B. nach 90 Min. zu einem Extinktionsmaximum kommt, man kann die monochromatische 388 nm Strahlung in einem Zweistrahlphotometer gleichzeitig als Meßstrahlung verwenden und registrieren (Fig. 6) (Bandbreite z.B. 5 nm).

Neben der Extinktionsmaximumsmessung (s. Fig. 6) läßt sich die Konformationsmodulation zusätzlich durch Messung der Fluoreszenz (Registrieung des Anregungsspektrums) nachweisen: Durch die photochrome Beeinflussung der Wirkstoffgruppe kommt es in allen Bereichen der Anregungsspektrofluorimetrie zu einem Kurvenverlauf, der von der Nullprobe weitgehend verschieden ist. Diese UV-induzierten Systeme beeinflussen die chemotherapeutische Wirkung in situ bzw. in vivo. Diese UV-aktivierte Wirkstofflösung ist u.a. geeignet im klinischen Experiment nicht nur bei AIDS, sondern auch beim T-Zell-Lymphom zur Beeinflussung der T-Lymphozyten eingesetzt zu werden. Es ist bekannt, durch UV-Aktivierung von 8-MOP, das aus einem Furanring und Cumarin besteht, Einfluß auf CTCL zu nehmen. Der Aufwand ist allerdings sehr hoch (Leukapherese) während es bei der erfindungsgemäß hergestellten UV-aktivierten Verbindung nur nötig ist, eine übliche parenterale Medikation durchzuführen.

Allgemein ist festzustellen, daß die Holzextraktionsbedingungen einen großen Einfluß auf die molekulare Struktur der löslichen Polysaccharid-Lignineinheiten haben. Die molekulare Struktur der Matrix hängt dabei in vielfältiger Weise von Aus-gangsmaterial ab, also davon, welches Kernholz eines Baumes, ob Stroh oder Gräser Verwendung findet. Um Spuren von Pflanzen-schutzmitteln zu umgehen, müssen alte Bäume verwendet werden.

Dabei ist auch die Holzart (Nadelbaum, Laubbaum) und auch das als Ausgangsmaterial eingesetzte Pflanzenteil (Nußschalen, Pfirsichkerne, Weichholzteile) bestimmend; so ist bspw. die Bindung zwichen P und L bei Nußschalen erheblich fester als bspw. bei einem Weichholz).

Ferner ist für das Produkt die Methode der LD-PLP-Netzwerkextration wesentlich, dabei sind insbesondere geeignet: alkalische Extraktion; Behandlung mit heißem Wasser/Wasserdampf unter Druck (Autoklaven) oder auch Elektrolyse mit NaCl als Elektrolyt.
Die erfindungsgemäße Wirkstoffgruppe enthält sehr viele Struktureinheiten aus dem Bereich der Naturstoffe Arene und Kohlenhydrate.

Nachfolgend sollen nun mit Material des Beispiels 1 an verschiedenen Zellsystemen durchgeführte Untersuchungen aufgeführt werden.

### Test der Wirkstoffgruppe aus Beispiel 1 an HIV-infizierten Human-Lymphozyten

Als Testsystem wurden Lymphozyten aus den Nabelschnurblut von Neugeborenen verwendet, die 2 Tage mit 10 µg/ml Phyto-hämagglutinin (PHA) voraktiviert worden waren. Dieses Zellkultursystem ist sehr sensitives lymphozytäres System zur Untersuchung von HIV. Es wurden etwa 200.000 PHA-aktivierte Nabelschnurblutlymphozyten in 1 ml-Kulturen in Gegenwart der Testsubstanz mit dem HIV-2-Isolat HIV-2ROD infiziert (Endkonzentration ca. 200 Syncytien-bildende Einheiten/ml). Eine lichtmikroskopische Auswertung der infizierten Kulturen erfolgte am dritten Tag nach Infektion (Untersuchung auf Entstehung von virusinduzierten Zellfusionen, sog. Syncytien).

Die Wirkstoffgruppe wurde in Kulturmedium ohne anschließende Sterilfiltration gelöst. Es wurden Konzentrationen von 200 µg/ml, 100 µg/ml, 50 µg/ml, 20 mg/ml, 2 µg/ml und 0,2 µg/ml getestet. Es wurden zwei Chargen, nämlich A88-1 und A88-4 untersucht.

### Test der Charge A 88-1 des Beispiels 1 in 24-Loch-Platten auf Hemmung der Syncytienbildung

| Konz. | lichtmikroskopische Auswertung am Tag 3-4 nach Infektion | |
|---|---|---|
| µg/ml | Syncytienbildung | Verträglichkeit |
| 200 | - | gute Kolonien |
| 100 | - | gute Kolonien |
| 50 | - | gute Kolonien |
| 20 | + | gute Kolonien |
| 2 | ++ | gute Kolonien |
| 0,2 | ++ | gute Kolonien |
| pos. Kontrolle | +++ | |
| - keine Syncytienbildung + vereinzelte Syncytienbildung (1-10) ++ deutliche Syncytienbildung (10-50) +++ starke Syncytienbildung (>50) | | |

### Test der Charge A88-4 in 24- Loch-Platten auf Hemmung der Syncytienbildung

| Konz. | lichtmikroskopische Auswertung am Tag 3-4 nach Infektion | |
|---|---|---|
| µg/ml | Syncytienbildung | Viabilität |
| 200 | - | gute Kolonien |
| 100 | - | gute Kolonien |
| 50 | - | gute Kolonien |
| 20 | + | gute Kolonien |
| pos. Kontrolle | +++ | |

### Auswertung der Hemmung der Synzytienbildung mit Charge II

Zielzellen:periphere Lymphozyten (PBL)
Virus: HIV-2ROD, 10⁵ SFU/ml, ca 1.500.000 cpm/ml/90 min eingesetzte Endverdünnung 1:500

| Konz.(µg/ml) | Syncytienbildung am Tag | | |
|---|---|---|---|
| | 2 | 3 | 4 |
| 1 | + | + | + |
| 5 | + | + | + |
| 10 | +/- | +/- | +/- |
| 25 | - | - | - |
| 35 | - | - | - |
| 50 | - | - | - |
| 75 | - | - | -* |
| 100 | - | - | -* |
| 150 | zytotoxisch | | |

| | | | |
|---|---|---|---|
| *leichte Zytotoxität | | | |

Wie aus der Tabelle ersichtlich ist, war diese Charge oberhalb 10 µg/ml wirksam.

### Anti-HIV-Aktivität der Wirkstoffgruppe (LABOR II)

Eine erfindungsgemäßes Wirkstoffgruppe wurde in zwei Konzen-trationen 2 mg/ml und 16 mg/ml in vitro untersucht. Dabei wurde von Labor 1 das HIV-core-protein, p24 mit der Antigen-capture Technik nach einer Primärinfektion mit H9-Zellen überprüft.

Phosphonoameisensäure wurde als positive Kontrollsubstanz verwendet und auch verschiedene Konzentrationen von AZT und ddC untersucht. Bei diesem Versuch zeigten beide Proben antivirale Aktivität.

Labor 2 verwendet die Syncytienbildung nach einer Primärinfektion von MT-2-Zellen als Endpunkt.

### Testergebnisse:

| LABOR 1 | | | |
|---|---|---|---|
| Wirkstoff-Konz. ug/l | % Hemmung des p24 | | |
| | Adaptogen 2 mg/ml | Adaptogen 16 mg/ml | Foscarnet |
| 1 | <30 | 30 - 50 | |
| 4 | <30 | 50 - 75 | |
| 10 | | | 50-75 |
| 20 | 50 - 75 | > 75 | |
| 50 | | | >75 |
| 100 | >75 | > 75 | Toxisch |
| 500 | Toxisch | >75 | Toxisch |
| geschätzte EC 50 | 15-20 µg/ml | 1-2 µg/ml | < 10 µg/ml |
| Positive Kontrolle: AZT und Dideoxycytidin (ddC) wurden jeweils mit 1 - 10 µg/ml getestet und zeigten eine >75%-ige Hemmung der p24-Expression ohne Cytotoxizität. | | | |

| LABOR 2 | | |
|---|---|---|
| Wirkstoff-Konz. ug/l | % Inhibierung der Syncytienbildung | |
| | Adaptogen 2 mg/ml | Adaptogen 16 mg/ml |
| 0,1 | 33 | |
| 0,16 | | 14,5 |
| 1 | 50 | |
| 1,6j | | |
| 10 | 82 | |
| 16 | | 88 |
| 48 | | 100 |
| 100 | 100 | |
| 160 | | 100 |
| 500 | etwas toxisch | |
| EC 50 | 1 µg/ml | 1 µg/ml |
| Positive Kontrolle: ddA mit 0,25 (90%-ige Hemmung) und 2,5 µg/ml (100% Hemmung). | | |

Aus obigem ist ersichtlich, daß die erfindungsgemäße Wirkstoffgruppe den Angriff von HIV Viren auf Zellen offensichtlich hindert.

In curativen Dosen (ca 0,1 mg/kg Körpergewicht Mensch) wirkt die Wirkstoffgruppe auch bei Dauergabe nicht sensi-bilisierend, nicht toxisch und hat keine Nebenwirkungen. Die erfindungsgemäße Wirkstoffgruppe verfügt über hydro-phile Domänen, die eine gute Wasserlöslichkeit bedingen.

Selbstverständlich sind weitere Abwandlungen und Maßnahmen des erfindungsgemäßen Prinzips im Rahmen des dem Fachmann auf diesem Gebiet Geläufigen möglich, so bspw. durch Einsatz anderer Monomere auf der Oberfläche einer Matrix.

Demzufolge ist die erfindungsgemäße Wirkstoffgruppe für eine Langzeitmedikation geeignet; es tritt bei Langzeitgabe kein Wirkungsverlust auf; es kann auch bei Verdachtsfall ohne Risiko eingesetzt werden. Allgemein zeigt es eine restitutive Wirkung und ist problemlos injizierbar. Aus den obigen Versuchen zeigt sich, daß die erfindungsgemäße Wirkstoffgruppe also signifikante antivirale Eigenschaften besitzt.

### Testreihe I (Survival-Effekt-Nachweis ohne adjuvante zelluläre Belastung)

Die erfindungsgemäße Wirkstoffgruppe besitzt aber auch kontraeskalative (restitutive) Eigenschaften, wie nachstehend ersichtlich. Akute Störungen im Bereich des Zentralnervensystems, wie sie zum Beispiel bei Trauma, Hirnverletzungen, Ischämie etc. auftreten, sind meist von Degeneration bzw. Zelltod zentraler Neuronen begleitet. Ebenso sind zahlreiche neu-rologische und neurodegenerative Erkrankungen des ZNS (z. B. Parkinson, Alzheimer, Korsakoff Syndrom) charakterisiert durch das Absterben selektiver Neuronen-Populationen.

Deshalb kommt der Untersuchung des Zelltodes bzw. des Überlebens von entralen Neuronen große Bedeutung zu. In den letzten Jahren hat die Forschung über neurotrophe Faktoren und über die Neuron-Glia-Wechselwirkung neue Erkenntnisse zu den Bedingungen des Überlebens von Neuronen in-vivo und in-vitro gesammelt.Dazu gehören auch neue Ergebnisse zur Bedeutung von Membran-Lipiden - unter Ein-schluß der Ganglioside und Phospholipide - für die Lebensfähigkeit von Nervenzellen. Experimente an Nervenzellkulturen zeigen, daß Peroxide bzw. Sauerstoff-Radikale durch Angriff der Membran-Lipide eine mögliche Rolle für die Überlebensfähigkeit ("Survival") bzw. den Zelltod spielen können.

### Material und Methoden

### I. Präparation der Hippocampus-Neuronen-Kultur

Die Hippocampi 17 Tage alter Rattenembryonen werden durch eine Trypsin/EDTA Behandlung und mechanisches Scheren dissoziiert und auf polylysin-beschichtete Coverslips ausplattiert. Nach einer Anheftungsphase in 10% Serum wachsen die Zellen in einem definierten, serumfreien Medium.

### II. Fluoreszenztest

Zwei Fluoreszenzfarbstoffe werden kombiniert eingesetzt, um lebende und tote Zellen unterschiedlich anzufärben, FluoreszeinDiaetat (FDA) wird als nicht fluoreszierende Vorstufe nur von lebenden Zellen aufgenommen, hydrolytisch gespalten und dadurch in eine fluoreszierende Verbindung (Fluoreszein) umgewandelt. Ethidiumbromid (EtBr=internationaler Freiname für 3,8-Diamino-5-ethyl-6-phenylphenanthridiniumbromid) bleibt von lebenden Zellen ausgeschlossen und dringt nur in tote Zellen ein, wo es den Kern in der Fluoreszenz rot darstellt.

### Medien

DMEM (Dulbeccos Modified Eagles Medium), Seromed, München
FKS (Fetales Kälberserum), Seromed, München 10 % in DMEM (1/2 h bei 56°C inaktiviert)

| HM (Hormon-Mix) | |
|---|---|
| Insulin | 8,8x10⁻⁷ M |
| Transferrin | 1,1x10⁻⁸ M |
| Trijodothyronin | 3x10⁻¹⁰ M |
| Hydrocortison in DMEM | 2x10⁻⁸ M |

### Wirkstoffkonzentrationen

In der Ampulle befinden sich 2mg/ml Lösung

| Verdünnung mit DMEM | | Endkonz. im Medium (100µl/2ml) | |
|---|---|---|---|
| 1:10 | 200µg/ml | 10µg/ml | (1) |
| 1:100 | 20µg/ml | 1µg/ml | (2) |
| 1:1000 | 2µg/ml | 100ng/ml | (3) |
| 1:10 000 | 200ng/ml | 10ng/ml | (4) |
| 1:100 000 | 20ng/ml | 1ng/ml | (5) |

Die Lösung wird den angehefteten Zellen in den Konzentrationen (1) - (5), nach der Anheftungsphase dem Medium zugegeben. Vitaltest nach 48 Stunden. Von jeweils 3 Kulturen pro Bedingung werden je 15 Felder mit ca. 50 Neuronen/Feld ausgezählt.

### Ergebnis

Der Vitaltest zeigt einen positiven Einfluß in einem Konzentrationsbereich um 100ng/ml Medium auf das Überleben der Neuronen in Kultur im Vergleich zur Kontrolle. Bei 10µg/ml zeigt sich ein leichter toxischer Effekt.

Bei der Beurteilung des "Survival"-Effekts muß bedacht werden, daß die Zellen unter optimierten Bedingungen wachsen, d.h., daß eine wesentliche Verbesserung des Überlebens nicht erwartet werden kann. Deshalb liegt es nahe, Experimente unter suboptimalen Bedingungen durchzuführen (Testreihe II).

### Vitaltest Hipp. Neuronen nach 48 Stunden in-vitro

### Verhältnis lebende/tote Zellen

### Testreihe II

### Einfluß auf das Überleben von Neuronen nach Schädigung

Bei mikrokinematographischen Aufnahmen wurde beobachtet, daß Zellen, die im Gesichtsfeld einer längeren Belichtung durch das Licht einer Halogenlampe ausgesetzt waren, früher absterben als jene ausserhalb.

Bei der Suche nach der Ursache für diesen Effekt haben wir in der Literatur Hinweise gefunden, daß in Zellkultur-Medien, die dem Licht von sog. Tageslicht-Lampen (mit einem hohen Anteil an kurzwelligem Licht), letale Photoprodukte gebildet werden. Bei der Bestrahlung von Tryptophan bzw. Tryptophan/Riboflavin-haltigen Medien mit nahem UV (365nm) entsteht in toxischen Mengen von H₂O₂ (McCormick, J.P., Fischer, J.R., Pachlatko, J.P. and Eisenstark, Science 191, 468-469 (1976); sowie Wang, R.J. and Nixon, B.T. In Vitro 14, No.8 19-22 (1978).

Peroxide und Sauerstoff-Radikale sind heute im Zusammenhang mit Alterungsprozessen von Zellen und Zell-Schädigungen durch toxische Einflüsse (z. B. infolge von Hypoxie) ins Blickfeld geraten.

Die folgende Studie untersucht den Einfluß des Wirkstoffs auf diesen zellschädigenden Effekt nach Belichtung mit Halogenlampenlicht.

In einem Vorversuch wurde zunächst die Dauer der Belichtung bestimmt, die zu einer deutlichen Verminderung des Überlebens der Zellen führt, aber noch nicht vollständig letal ist.

### Methode:

Neuronen aus dem Hippocampus von Rattenembryonen (Tag 17), wurden - wie beschrieben - präpariert. Nach 3 bis 4 Stunden Auswachsen im Brutschrank, wurden die Kulturschalen herausgenommen und einer Belichtung ausgesetzt. Der Wirkstoff wurde anschließend in 3 Konzentrationsstufen mit frischem Medium den Kulturen zugegeben und diese 20 Stunden weiterkultiviert. Das Überleben der Zellen wurde mit den beschriebenen Fluoreszenztest bestimmt. Als Kontrolle dienten belichtete sowie nicht belichtete Zellen, denen frische Medium ohne Wirkstoff zum gleichen Zeitpunkt zugegeben wurde.

### Belichtung:

500W Halogenlampe mit Reflektor, Abstand 40 cm, (Lichtwert am Ort der Petrischalen gemessen: LW 18,5), Belichtungsdauer 20min. Die Kulturschalen befinden sich während der Belichtung in temperiertem Wasserbad, wobei die Temperatur direkt im Medium gemessen, 34°C nicht übersteigt.

### Ergebnis:

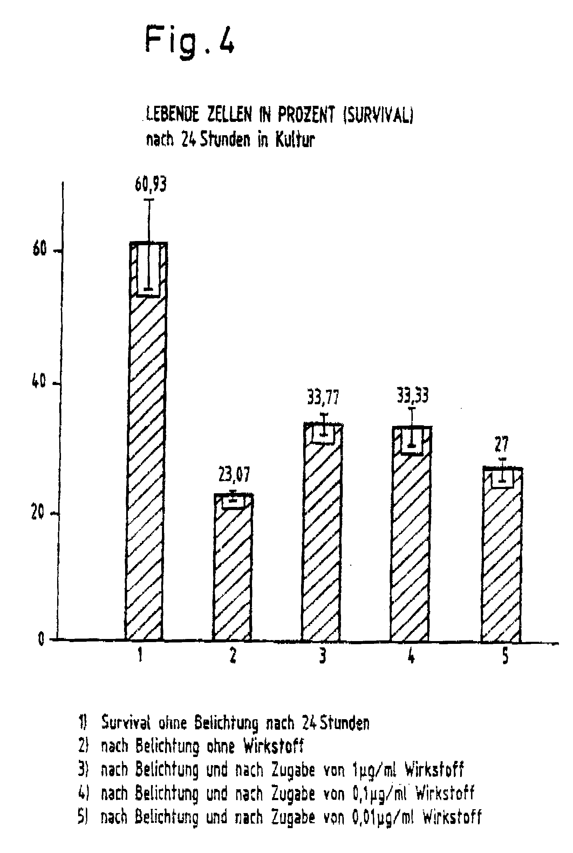

Das Ergebnis ist in Fig. 4 dargestellt, woraus sich ergibt: Lebende Zellen in Prozent (SURVIVAL) nach 24 Stunden in Kultur
1) Survival ohne Belichtung nach 24 Stunden
2) nach Belichtung ohne Wirkstoff
3) nach Belichtung und nach Zugabe von 1 µg/ml Wirkstoff
4) nach Belichtung und nach Zugabe von 0,1 µg/ml Wirkstoff
5) nach Belichtung und nach Zugabe von 0,01 µg/ml Wirkstoff
Es zeigt sich ein deutlicher Effekt auf das Überleben der Neuronen in Kultur unter den beschriebenen Bedingungen, wobei auffällt, daß eine Konzentration (1 µg/ml) wirksam ist, bei der unter optimalen Bedingungen nach 48 Stunden kein Survival-Effekt zu beobachten ist (siehe Bericht I). Möglicherweise überlagert unter Normalbedingungen ein leichter toxischer Effekt die protektive Wirkung, die nach Schädigung durch Licht überwiegt.

Nicht nachgegangen wurde der Frage, welcher Teil des Spektrums für die letalen Effekte am wirksamsten ist. Es wurde aber ausgeschlossen, daß sich das Medium durch den hohen langwelligen Anteil des Lichtes aufheizt und evtl. dadurch die Zellen geschädigt werden.

## Patentansprüche

1. Wirkstoffgruppe zur restitutiven Chemotherapie bei viralen Infektionskrankheiten, erhältlich durch
I) Herstellung polysaccharidreicher Lignin-Einheiten (PLP) durch:
Extraktion von Holz oder holzartigen Materialien auch aus (oder) pflanzlichen Zellkulturen (A) in wässrigem Medium in schwach saurem bis alkalischem Milieu;
Abtrennen der unlöslichen festen Bestandteile;
II) Herstellen niedermolekularer polysaccharidarmer lignoider Einheiten (LPL) durch:
wässrig-alkalische Extraktion (pH 7 - 14) von Holz-Inkohlungsprodukten oder biokonvertierten holzartigen Materialien (B);
Abtrennen der alkaliunlöslichen festen Bestandteile;
III) Herstellung eines wasserlöslichen Umsetzungsprodukts LD(PLP=LPL) mit niedrigem Molekulargewicht (LD) aus den Produkten von I) und II) durch:
Umsetzung des Produktes des Schrittes I, den polysaccharidreichen Lignineinheiten (PLP) mit den Produkten des Schrittes II, den polysaccharidarmen lignoiden Einheiten (LPL) unter wässrig-alkalischen Bedingungen (pH 8-14);
Isolation der Low-Dalton-Fraktionen dieses Umsetzungsprodukts LD(PLP=LPL) durch Ultrafiltration bei nominellen Trennschnittwerten von etwa 15 bis 40 kD, wobei das Retentat verworfen wird;
Behandlung der Lösung mit H⁺-Kationenaustauscher bei pH 3 bis 7,0, sowie Weiterverarbeitung der sauren Lösung der Wirkstoffgruppe LD(LPL=PLP).

2. Wirkstoffgruppe nach Anspruch 1, dadurch gekennzeichnet, daß man in Schritt I) polysaccharid-reiche Lignin-Einheiten HD(PLP) mit weniger als 100 kD gewinnt, indem nach der Extraktion in wässrigen Medien und Abtrennen der unlöslichen festen Bestandteile eine Einstellung des pH-Wertes auf neutral bis schwach sauer erfolgt;
eine Molekularfiltration, bevorzugt Ultrafiltration bei einem Trennschnittwert von kleiner als 100 kD; bevorzugt kleiner als 70 kD, erfolgt und das Retentat verworfen wird;
die derart erhaltene Lösung von Substanzen mit niedrigem Dalton wird in Schritt III) weiterverarbeitet wird.

3. Wirkstoffgruppe nach Anspruch 1 oder 2, dadurch gekennzeichnet, daß bei Schritt II niedermolekulare polysaccharidarme lignoide Einheiten LD-LPL mit weniger als etwa 40 kD hergestellt werden, indem nach der wässrig-alkalischen Extrak-tion und Abtrennen der alkaliunlöslichen festen Bestandteile eine alkalische Behandlung bei pH-Werten über 8 durchgeführt wird und danach eine Molekulartrennung durch Ultrafiltration bei nominellem Trennschnitt von 15 bis 35 kD erfolgt und das so erhaltene Produkt mit niedrigem Dalton in Schritt III weiterverarbeitet wird.

4. Wirkstoffgruppe nach einem der vorangehenden Ansprüche, dadurch gekennzeichnet, daß ggf. nach der Umsetzung der polysaccharidreichen Produkte des Schrittes I (PLP) mit den polysaccharidarmen Fraktionen (LPL) eine Feststofftrennung durchgeführt wird und die verbleibende Lösung weiterverarbeitet wird.

5. Wirkstoffgruppe nach einem der vorangehenden Ansprüche, dadurch gekennzeichnet, daß die Weiterverarbeitung der aus Schritt III erhaltenen sauren LD(LPL=PLP)-Lösung der Wirkstoffgruppe durch Partikelfilterung und Pyrogenentfernung durch Filterung und anschließender Sterilisation sowie ggf. Konfektionierung erfolgt.

6. Wirkstoffgruppe nach einem der vorangehenden Ansprüche, dadurch gekennzeichnet, daß die zu extrahierenden holzartigen Materialien in Schritt I) ausgewählt sind aus der Gruppe bestehend aus::
A1: nativem Holz
A2: Holz-Analoga; biotechnologisch darstellbaren holzigen Substanzen durch Aufbereitung (Züchtung) pflanzlicher Zellkulturen
A3: synthetischen Holz-Analoga nach Darstellung von FREUDENBERG-Dehydrierungspolymerisaten (DHP) von Mono- und/oder Dilignolen und Aufpfropfen der DHP's auf Polysaccharide;
A4: Lignin-Polysaccharid-Komplexe, erhältlich durch alkalische Extraktion von Chlorit-Holzzellulose);
sowie Mischungen (A1, A2, A3, A4) derselben.

7. Wirkstoffgruppe nach einem der vorangehenden Ansprüche, dadurch gekennzeichnet, daß die in Schritt II) verwendeten pflanzlichen Ausgangsmaterialien ausgewählt sind aus der Gruppe bestehend aus:
B1: nativen Produkten der Inkohlung
B2: durch lignolytische aktive Mikroorganismen, Weißfäulepilze, biokonvertiertes Holz
B3: durch Einwirkung isolierter lignolytischer Enzyme (wie Phenoloxidase) biokonvertiertem Holz,
oder Mischungen (B1, B2, B3) derselben;

8. Wirkstoffgruppe nach einem der vorangehenden Ansprüche, dadurch gekennzeichnet, daß die Basen KOH, NaOH, LiOH und Ammoniak sind.

9. Wirkstoffgruppe nach einem der vorangehenden Ansprüche, dadurch gekennzeichnet, daß die in Schritt I gewonnenen polysaccharidreichen Lignin-Einheiten (PLP) in einer Konzentration von 0,05 - 10 Gew%; bevorzugt etwa bis 2 Gew.% und ganz besonders bevorzugt in etwa 0,1 Gew.% in wäßriger Lösung vorliegen.

10. Wirkstoffgruppe nach einem der vorangehenden Ansprüche, dadurch gekennzeichnet, daß in Schritt I für das zu gewinnende polysaccharidreiche Produkt PLP bei einem pH-Wert von etwa 12 - 14, bevorzugt in KOH über 1 - 10 Tage bei Raumtemperatur (20 °C) extrahiert wird.

11. Wirkstoffgruppe nach einem der vorangehenden Ansprüche, dadurch gekennzeichnet, daß das polysaccharidreiche Extraktionsprodukt PLP durch Extraktion bei erhöhter Temperatur bis zu 120 °C, wobei bei Temperaturen über etwa 100 °C im Autoklaven gearbeitet wird, gewonnent wird.

12. Wirkstoffgruppe nach einem der vorangehenden Ansprüche, dadurch gekennzeichnet, daß die Holz-Inkohlungsprodukte ausgewählt sind aus der Gruppe bestehend aus Ligniten, Farbkohlen, Braunkohle.

13. Wirkstoffgruppe nach einem der vorangehenden Ansprüche, dadurch gekennzeichnet, daß die alkalische Extraktion des Schrittes II mit 0.1-0,8 M KOH bei Raumtemperatur, bevorzugt mit 0,4 M KOH, erfolgt.

14. Wirkstoffgruppe nach einem der vorangehenden Ansprüche, dadurch gekennzeichnet, daß die alkalische Autofragmentierung innerhalb von 7 bis 10 Tagen bei pH 11 (KOH) bei Raumtemperatur erfolgt.

15. Wirkstoffgruppe nach einem der vorangehenden Ansprüche, dadurch gekennzeichnet, daß die Umsetzung zum Produkt PLP=LPL des Schrittes III bei einer Temperatur zwischen Raumtemperatur und 120°C und bei einem pH-Wert von 8-14, bevorzugt 10-12, erfolgt.

16. Wirkstoffgruppe nach einem der vorangehenden Ansprüche, dadurch gekennzeichnet, daß das Produkt des Schrittes III LD(PLP=LPL) bei Raumtemperatur einer Molekulartrennung zur Gewinnnung einer Low-Dalton-Fraktion unterwofen wird, bevorzugt einer Niederdruck-Ultrafiltration an alkalifesten Ultrafiltrations-Membranen mit einem nominellen Trennschntt zwischen 18 - 38 kD.

17. Wirkstoffgruppe nach einem der vorangehenden Ansprüche, dadurch gekennzeichnet, daß das Low-Dalton-Produkt des Schrittes III LD(PLP=LPL) einem Austausch der Alkaliionen durch Kunst-harzkationenaustauscher in der Wasserstofform unterworfen wird, bis zu einem stabilen pH-Wert im Bereich von 4 bis 6,8, bevorzugt 5 bis 6.

18. Wirkstoffgruppe nach einem der vorangehenden Ansprüche, dadurch gekennzeichnet, daß das saure Produkt des Schrittes III LD-(PLP=LPL) nach der Kationenaustauscherbehandlung durch Filterung von Pyrogenen befreit und anschließend durch Behandlung im Autoklaven, bspw. bei 121 °C über 14 Minuten sterilisiert wird.

19. Wirkstoffgruppe nach einem der vorangehenden Ansprüche, dadurch gekennzeichnet, daß die wäßrige Lösung des Umsetzungsprodukts des Schrittes III, der Wirkstoffgruppe LD(PLP=LPL) in an sich bekannter Weise, ggf. mit Hilfsstoffen, zu einer fließfähigen pharmazeutischen Zubereitung verarbeitet wird.

20. Wirkstoffgruppe nach einem der vorangehenden Ansprüche, dadurch gekennzeichnet, daß die wäßrige Lösung der Wirkstoffgruppe LD(PLP=LPL) in an sich bekannter Weise in Ampullen für Injektionszwecke abgefüllt wird.

21. Wirkstoffgruppe nach einem der vorangehenden Ansprüche zur Bekämpfung viraler Infektionen.

22. Wirkstoffgruppe nach Anspruch 21, dadurch gekennzeichnet, daß sie in parenteraler oder lokaler Applikationsform vorliegt.

23. Wirkstoffgruppe nach Anspruch 21 zur Bekämpfung von Retroviren, insbesondere vom HIV-Typ, auch AIDS-Viren.

24. Wirkstoffgruppe nach einem der Ansprüche 21 und 23, dadurch gekennzeichnet, daß sie chelatisiertes Pt, Au, Pd aufweist.

## Claims

1. Active substance group for use in restitutive chemotherapy against viral infections. The system is obtained as follows:
I) Preparation of lignin units high in polysaccharides (PLP) by:
Extraction in aqueous media, under weakly acid or alkaline conditions, from wood or wood-like materials and/or from plant-cell cultures (A);
separating off the insoluble solids;
II) Preparation of low-molecular-weight lignoid units low in polysaccharides (LPL) by:
Aqueous alkaline extraction (pH 7-14) from wood-incarbonization products or bioconverted wood-like materials (B);
separating off the alkali-insoluble solids:
III) Preparation of a water-soluble reaction product LD(PLP=LPL) with low molecular weight (LD) from the products of I) and II) by:
Reacting the product of step (I), the lignin units high in polysaccharides (PLP), with the products of step (II), the lignoid units low in polysaccharides (LPL), under aqueous alkaline conditions (pH 9-12);
Isolation of the LD fractions of this HD(PLP=LPL) reaction product by ultrafiltration, taking the cut between the nominal values of about 15 to 40 kD, and discarding the residue;
Treatment of the solution with H+ cation exchanger at pH 3 to 7.0 and processing the acid solution of the active substance group LD(LPL=PLP).

2. Active substance group in accordance with claim 1, characterized by the following: In step (I) lignin units high in polysaccharides HD(PLP) are prepared with less than 100 kD by: Setting the pH value between neutral and weakly acid after extraction in aqueous media and separating off the insoluble solids; a molecular filtration, preferably ultrafiltration with a suitable cut of less than 100 kD, preferably less than 70 kD, is carried out and the residue is discarded.
The solution obtained in this way is processed in step (III).

3. Active substance group in accordance with claim 1 or 2, characterized by the following: In step II, low-molecular-weight lignoid units low in polysaccharides LD-LPL are prepared with less than about 40 kD as follows: After the aqueous alkaline extraction and separating off of alkaline-insoluble solids, an alkaline fragmentation with pH values over 8 and molecular separation by ultrafiltration with nominal cut of 15 to 35 kD follows and the product obtained in this way is processed in step III.

4. Active substance group in accordance with the above claims, characterized by the following: If necessary, after reacting the high polysaccharide with the low polysaccharide fractions (i.e. the intermediates of step I and step II), a separation of the solids is carried out and then the remaining solution is processed further.

5. Active substance group in accordance with the above claims, characterized by the following: The further processing of the acid LD(LPL-PLP) solution obtained from step III is carried out by means of particle filtering, pyrogen removal via filtering, and then sterilization and if required, manufacturing.

6. Active substance group in accordance with the above claims, characterized by the following: The wood-like materials used for extraction in step I) are selected from the group consisting of:
A1: Native wood
A2: Wood analogues like woody substances biotechnologically produced by the preparation (growing) of plant-cell cultures.
A3: Synthetic wood analogues via the production of FREUDENBERG dehydration polymers (DHP) from mono- and/or dilignoles and grafting the DHP's on polysaccharide;
A4: Lignin-polysaccharide complexes (networks) obtained by alkaline extraction from chlorite wood cellulose;
as well as mixtures (A1, A2, A3, A4) of these.

7. Active substance group in accordance with the above claims, characterized by the following: The initial materials used in step II) selected from the group consisting of:
B1: Native products of incarbonization
B2: Wood bioconverted by lignolytically active micro-organisms like white wood-putrefying fungi.
B3: Wood bioconverted through the effect of isolated lignolytic enzymes (like phenoloxidase),
or mixtures (B1, B2, B3) of these.

8. Active substance group in accordance with the above claims, characterized by the following: The bases are KOH, NaOH, LiOH, and ammonia.

9. Active substance group in accordance with the above claims, characterized by the following: The lignin units high in polysaccharides (PLP) obtained in step 1 occur in a concentration of 0.05 - 10 % by weight; preferably up to about 2 % by weight and most preferably at about 0.1 % by weight.

10. Active substance group in accordance with the above claims, characterized by the following: In step 1 for the PLP product high in polysaccharides which is to be obtained, an extraction is carried out under a pH value of approx. 12-14, preferably in KOH over 1 - 10 days at room temperature (20°C).

11. Active substance group in accordance with the above claims, characterized by the following: The PLP extraction product is produced via extraction under increased temperature up to 120 °C, with processing at temperatures of over approx. 100 °C being carried out in an autoclave.

12. Active substance group in accordance with the above claims, characterized by the following: The wood incarbonization products are selected from the group consisting of lignites, coloured coals, and brown coal.

13. Active substance group in accordance with the above claims, characterized by the following: The alkaline extraction with 0.1-0.8 M KOH, preferably with 0.4 M KOH, occurs at room temperature.

14. Active substance group in accordance with the above claims, characterized by the following: The alkaline fragmentation is carried out over 7 to 10 days at pH 11 (KOH) at room temperature.

15. Active substance group in accordance with the above claims, characterized by the following: The conversion to product of step III, PLP=LPL, occurs at a temperature between room temperature and 120°C and under a pH value of 8-14, preferably 10-12.

16. Active substance group in accordance with the above claims, characterized by the following: The product of step III, LD(PLP=LPL), is subjected to molecular separation to obtain an LD fraction, preferably by means of a low-pressure ultrafiltration through alkali-stable ultrafiltration membranes with a nominal cut between 18 and 38 kD.

17. Active substance group in accordance with the above claims, characterized by the following: The low-dalten product of step III, LD (PLP=LPL), is subjected to an exchange of the alkali ions by means of an artificial resin cation exchanger in the hydrogen form until reaching a stable pH value in the area of 4 to 6.8, preferably 5-6.

18. Active substance group in accordance with the above claims, characterized by the following: After the cation exchange treatment, the acidic product of step III, LD (PLP=LPL), has the pyrogens removed by filtering and then, for example, is sterilized by means of autoclaving at, for example, 121°C over 14 min.

19. Active substance group in accordance with the above claims, characterized by the following: The aqueous solution of the reaction product of step III, (PLP=LPL), is processed in the usual way, if necessary with inactive ingredients, to a fluid pharmaceutical preparation in accordance with the form of administration.

20. Active substance group in accordance with the above claims, characterized by the following: The aqueous solution of the active substance group LD(PLP=LPL) is processed in the usual way to ampoules for injection purposes.

21. Active substance group in accordance with the above claims for the combating of viral infections.

22. Active substance group in accordance with claim 21, characterized by the following: They are available in parenteral or local application forms.

23. Active substance group in accordance with claim 21 used for the compating of retroviruses, particularly of the HIV type, also AIDS viruses.

24. Active substance group in accordance with claims 21 and 23 characterized by chelated Pt, Au, or Pd.

## Revendications

1. Groupe de principes actifs pour chimiothérapie de restitution dans le cas de maladies infectieuses virales, susceptible d'être obtenu par :
I) préparation d'unités de lignine riches en polysaccharides (PLP) par :
extraction à partir de bois ou de matières ligneuses et/de cultures cellulaires végétales (A) en milieu agueux faiblement acide à alcalin,
séparation des composants solides insolubles,
II) préparation d'unités lignoïdes pauvres en polysaccharides de faible poids moléculaire (LPL) par :
extraction en milieu aqueux alcalin (pH 7 - 14) à partir de produits de carbonisation du bois ou de matières ligneuses bioconverties (B),
séparation des composants solides insolubles dans l'alcali,
III) préparation d'un produit réactionnel soluble dans l'eau LD (PLP=LPL) de faible poids moléculaire (LD) à partir des produits de I) et de II) par :
réaction du produit du stade I, les unités de lignine en polysaccharides (PLP), avec les produits du stade II, les unités lignoïdes pauvres en polysaccharides (LPL) en milieu aqueux alcalin (pH 8 - 14),
isolement des fractions de faible poids atomique dalton de ce produit réactionnel LD (PLP=LPL) par ultrafiltration à des valeurs nominales de la couche de séparation d'environ 15 à 40 kd, le rétentat étant mis au rebut,
traitement de la solution par un échangeur de cations H⁺ à pH 3 à 7,0 ainsi que transformation de la solution acide du groupe de principes actifs LD (LPL=PLP).

2. Groupe de principes actifs selon la revendication 1, caractérisé en ce que l'on obtient au stade I) des unités de lignine riches en polysaccharides HD (PLP) de moins de 100 kd en réglant la valeur du pH à une valeur neutre à faiblement acide après l'extraction dans des milieux aqueux et la séparation des composants solides insolubles,
on effectue une filtration moléculaire, de préférence une ultrafiltration à une valeur de la couche de séparation de moins de 100 kd, de préférence de moins de 70 kd, et on met au rebut le rétentat;
on transforme la solution ainsi obtenue de substances de faible poids atomique dalton au stade III).

3. Groupe de principes actifs selon la revendication 1 ou 2, caractérisé en ce que les unités lignoïdes LD-LPL pauvres en polysaccharides de faible poids moléculaire, d'un poids atomique de moins d'environ 44 kd, sont préparées au stade II, en réalisant, après l'extraction en milieu aqueux alcalin et la séparation des composants solides insolubles dans les alcalis, un traitement alcalin à des valeurs du pH supérieures à 8 et ensuite on effectue une séparation moléculaire par ultrafiltration a une valeur nominale de la couche de séparation de 15 à 35 kd et on transforme le produit ainsi obtenu de faible poids atomique dalton au stade III.

4. Groupe de principes actifs selon l'une quelconque des revendications précédentes, caractérisé en ce qu'on réalise éventuellement après la réaction des produits riches en polysaccharides du stade I (PLP) et des fractions pauvres en polysaccharides (LPL), une séparation des matières solides et on transforme la solution qui subsiste.

5. Groupe de principes actifs selon l'une quelconque des revendications précédentes, caractérisé en ce que la transformation de la solution acide LD (LPL=PLP) du groupe de principes actifs obtenue au stade III se fait par filtration à travers des particules et élimination des pyrogènes par filtration et ensuite par stérilisation ainsi qu'éventuellement par conditionnement.

6. Groupe de principes actifs selon l'une quelconque des revendications précédentes, caractérisé en ce que les matières ligneuses à extraire au stade I sont choisies dans le groupe constitué des matières suivantes :
A1 : bois natif
A2 : analogues du bois; substances ligneuses représentables par biotechnologie par préparation (culture) de cultures cellulaires végétales
A3 : analogues du bois synthétiques selon la représentation de FREUDENBERG de polymérisats de déshydratation (DHP) de mono et/ou dilignols et greffe des DHP sur des polysaccharides,
A4 : complexes de lignine et de polysaccharides susceptibles d'être obtenus par extraction alcaline de cellulose de bois et de chlorite,
ainsi que des mélanges (A1, A2, A3, A4) de ces substances.

7. Groupe de principes actifs selon l'une quelconque des revendications précédentes, caractérisé en ce que les matières végétales de départ utilisées au stade II sont constituées du groupe suivant :
B1 : produits natifs de carbonisation
B2 : via des micro-organismes actifs lignolytiques, des moisissures de pourritures alvéolaires, du bois bioconverti,
B3 : par action d'enzymes lignolytiques isolés (comme la phénoloxydase), du bois bioconverti,
ou des mélanges (B1, B2, B3) de ces substances.

8. Groupe de principes actifs selon l'une quelconque des revendications précédentes, caractérisé en ce que les bases sont du KOH, du NaOH, du LiOH ou de l'ammoniac.

9. Groupe de principes actifs selon l'une quelconque des revendications précédentes, caractérisé en ce que les unités de lignine riches en polysaccharides (PLP) obtenues au stade I sont présentes en concentration de 0,05 à 10 % en poids, de préférence jusqu'à environ 2 % en poids et mieux encore jusqu'à environ 0,1 % en poids en solution aqueuse.

10. Groupe de principes actifs selon l'une quelconque des revendications précédentes, caractérisé en ce que les produits riches en polysaccharides PLP susceptibles d'être obtenus au stade I sont extraits à pH d'environ 12 - 14, de préférence dans du KOH, sur une période de 1 à 10 jours à température ambiante (20°C).

11. Groupe de principes selon l'une quelconque des revendications précédentes, caractérisé en ce que le produit d'extraction riche en polysaccharides PLP est obtenu par extraction à température élevée jusqu'à 120°C, en opérant notamment dans des autoclaves à des températures supérieures à environ 100°C.

12. Groupe de principes actifs selon l'une quelconque des revendications précédentes, caractérisé en ce que les produits de carbonisation du bois sont choisis dans le groupe formé des lignites, des charbons colorés et de la lignite.

13. Groupe de principes actifs selon l'une quelconque des revendications précédentes, caractérisé en ce que l'extraction alcaline du stade II se fait avec 0,1 à 0,8 M KOH à température ambiante, de préférence avec 0,4 M KOH.

14. Groupe de principes actifs selon l'une quelconque des revendications précédentes, caractérisé en ce que l'autofragmentation alcaline se fait en l'espace de 7 à 10 jours à pH de 11 (KOH) à température ambiante.

15. Groupe de principes actifs selon l'une quelconque des revendications précédentes, caractérisé en ce que la conversion en produit PLP=LPL du stade III se fait à une température comprise entre la température ambiante et 120°C et une valeur du pH de 8 - 14, de préférence, de 10 - 12.

16. Groupe de principes actifs selon l'une quelconque des revendications précédentes, caractérisé en ce que le produit du stade III LD (PLP=LPL) est soumis, à température ambiante, à une séparation moléculaire pour obtenir une fraction de faible poids atomique dalton, de préférence à une ultrafiltration sous dépression à travers des membranes d'ultrafiltration résistant aux alcalis et ayant une couche de séparation nominale comprise entre 18 et 38 kd.

17. Groupe de principes actifs selon l'une quelconque des revendications précédentes, caractérisé en ce que le produit de faible poids atomique dalton du stade III LD(PLP=LPL) est soumis à un échange d'ions alcalins à travers un échangeur de cations de résine synthétique sous la forme d'hydrogène jusqu'à obtenir une valeur du pH stable de 4 à 6,8, de préférence de 5 à 6.

18. Groupe de principes actifs selon l'une quelconque des revendications précédentes, caractérisé en ce que le produit acide du stade III LD (PLP=LPL) est débarrassé des pyrogènes par filtration après échange de cations et est ensuite stabilisé par traitement à l'autoclave, par exemple, à 121°C pendant 14 minutes.

19. Groupe de principes actifs selon l'une quelconque des revendications précédentes, caractérisé en ce que la solution aqueuse du produit réactionnel du stade III, le groupe de principes actifs LD (PLP=LPL), est traité de manière connue en soi, éventuellement avec des substances auxiliaires, pour obtenir une préparation pharmaceutique coulante.

20. Groupe de principes actifs selon l'une quelconque des revendications précédentes, caractérisé en ce que la solution aqueuse du groupe de principes actifs LD (PLP=LPL) est de manière connue en soi utilisée pour remplir des ampoules à des fins d'injection.

21. Groupe de principes actifs selon l'une quelconque des revendications précédentes pour lutter contre les infections virales.

22. Groupe de principes actifs selon la revendication 21, caractérisé en ce qu'il se présente sous une forme d'application parentérale ou locale.

23. Groupe de principes actifs selon la revendication 21 pour lutter contre les rétrovirus, en particulier du type HIV, ainsi que contre les virus du SIDA.

24. Groupe de principes actifs selon l'une quelconque des revendications 21 et 23, caractérisé en ce qu'il présente du Pt, de l'Au, du Pd chélaté(s).
